(19) Europäisches Patentamt
European Patent Office
Office européen des brevets

(11) **EP 1 144 408 B1**

(12) **FASCICULE DE BREVET EUROPEEN**

(45) Date de publication et mention de la délivrance du brevet:
**25.09.2002 Bulletin 2002/39**

(21) Numéro de dépôt: **00900639.6**

(22) Date de dépôt: **21.01.2000**

(51) Int Cl.[7]: **C07D 417/12**, C07D 413/12, A61K 31/54, A61K 31/535

(86) Numéro de dépôt international:
**PCT/FR00/00137**

(87) Numéro de publication internationale:
**WO 00/043391 (27.07.2000 Gazette 2000/30)**

(54) **N-(PIPERIDIN-4-YL)-4H-3,1-BENZO(THIA/OXA)ZINES-2-AMINES SUBSTITUEES, LEUR PREPARATION ET LEUR APPLICATION EN THERAPEUTIQUE**

SUBSTITUIERTE N-(PIPERIDIN-4-YL)-4H-3,1-BENZO(THIA/OXA)ZIN-2-AMINE, IHRE HERSTELLUNG UND THERAPEUTISCHE VERWENDUNG

SUBSTITUTED 1-(PIERIDIN-4-YL)-3-(ARYL) ISOTHIOUREAS THEIR PREPARATION AND THERAPEUTIC APPLICATION

(84) Etats contractants désignés:
**AT BE CH CY DE DK ES FI FR GB GR IE IT LI LU MC NL PT SE**

(30) Priorité: **22.01.1999 FR 9900706**

(43) Date de publication de la demande:
**17.10.2001 Bulletin 2001/42**

(73) Titulaire: **PIERRE FABRE MEDICAMENT**
**92100 Boulogne-Billancourt (FR)**

(72) Inventeurs:
- **RIEU, Jean-Pierre**
  **F-81100 Castres (FR)**
- **PATOISEAU, Jean-François**
  **F-81100 Castres (FR)**
- **JOHN, Gareth**
  **F-81100 Castres (FR)**
- **LEGRAND, Bruno**
  **F-81440 Lautrec (FR)**
- **VERSCHEURE, Yvan**
  **F-81100 Castres (FR)**

(74) Mandataire: **Ahner, Francis et al**
**Cabinet Régimbeau**
**20, rue de Chazelles**
**75847 Paris cedex 17 (FR)**

(56) Documents cités:
**WO-A-97/05134**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 144 408 B1

Printed by Jouve, 75001 PARIS (FR)

## Description

L'invention concerne de nouvelles N-benzo(thia/oxa)zines-2-yl-1-arylalcoyloxyalkyl-4-pipéridinamine substituées de formule I :

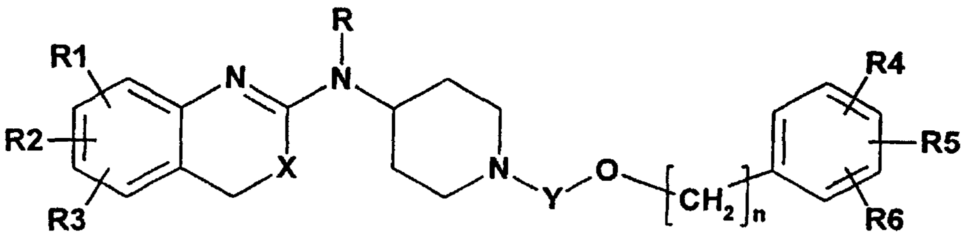

**( I )**

(où les radicaux sont tels que définis dans l'invention), leur préparation et leur utilisation en thérapeutique.

Dans un brevet récent de la demanderesse (WO-97.05134) il était fait état de N-alcoyl-N-[1-(ω-aryloxyalcoyl) pipéridin-4-]-4H-3,1-benzothiazin-2-amines possédant un intérêt thérapeutique, particulièrement dans le traitement de l'ischémie myocardique.

Au cours de l'étude de structure activité, il a été décidé d'augmenter l'espacement entre le radical phényle et l'oxygène par l'introduction d'un radical alcoylène pour mesurer l'impact sur l'activité cytoprotectrice d'une telle modification structurale. La substitution de l'azote pipéridinique par un radical arylacoyloxyalcoyl à la place d'un radical aryloxy alcoyl a permis d'identifier une nouvelle classe de composés qui ont montré une activité supérieure aux composés de la série de base. Les composés de cette nouvelle série font l'objet de la présente invention.

**MOLECULES REVENDIQUEES :**

Les molécules de la présente invention appartiennent à la classe des N-alcoyl-N-[1-($\bar{\omega}$-(arylalcoyloxy)alcoyl] pipéridin-4-yl]-4H-3,1-benzo(thia,xa)zines-2-amines substituées et ont pour **formule I :**

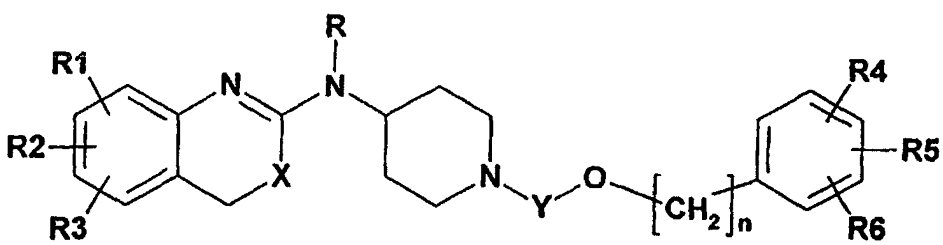

**( I )**

dans laquelle :

- **X** représente un atome d'oxygène ou de soufre
- **Y** représente soit
  - * un radical alcoylène ramifié ou non renfermant de 2 à 6 atomes de carbone ou
  - * le radical $-CH_2-CH(OH)-CH_2-$
- **R** représente un hydrogène, un radical alcoyle saturé ou non, ramifié ou non renfermant de 1 à 7 atomes de carbone,
- **$R_1$ à $R_6$** égaux ou différents représentent :

* un hydrogène,
* un alcoyle ramifié ou non, saturé ou non renfermant de 1 à 5 atomes de carbone,
* un alcoyloxy ramifié ou non saturé ou non renfermant de 1 à 5 atomes de carbone,
* un groupement halogéno,
* un groupement nitro,
* un groupement hydroxy,
* un groupement acyle ou acyloxy renfermant de 2 à 3 atomes de carbone,
* un groupement dialcoylamino renfermant de 1 à 5 atomes de carbone,
* un groupement trifluorométhyle ou trifluorométhoxyle.

**n** est un nombre entier pouvant varier de 1 à 6 inclus.

La présente invention inclut aussi les sels minéraux ou organiques thérapeutiquement acceptables des composés de formule I et leurs hydrates éventuels. Elle concerne aussi les procédés de préparation des dérivés revendiqués ainsi que leur application comme médicaments. Les molécules de la présente invention de formule I possèdent des propriétés cytoprotectrices remarquables, d'activité supérieure à celle de la famille de base revendiquée dans le brevet WO 97.05134.

**<u>SYNTHESE DES COMPOSES DE FORMULE I</u> :**

Les composés de formule I peuvent être préparés selon deux voies principales, en terminant soit par la N-alcoylation soit par l'hétérocyclisation.

**1°) Synthèse par N-alcoylation finale :**

**a**) Dans le **cas ou Y représente un alcoylène,** les composés de formule **I** sont généralement préparés par condensation d'un halogénure ou un mésylate d'arylalcoyloxyalcoyle de formule **III** sur une N-(pipéridin-4-yl)-4H-3,1-benzothiazine-2-amine **II** en présence d'une base organique ou minérale dans un solvant organique selon la réaction :

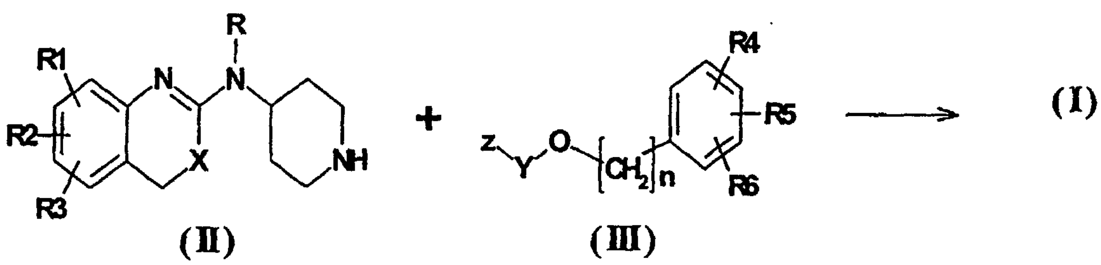

avec Z = Cl, Br, I, $MeSO_3$.

La méthode de préparation des éthers **III** varie selon la longueur de la chaîne carboné du groupement alcoylène divalent **Y**.

* Lorsque la chaîne de base **Y** renferme plus de 3 atomes de carbone, l'éther est préparé par condensation d'un excès de 1, ω-dibromoalcane **V** sur l'alcool correspondant **IV** en milieu basique fort en utilisant un catalyseur de transfert de phase comme l'hydrogéno sulfate de tétrabutylammonium selon **A.W. Burgstahler** et coll. (*J. Org. Chem.*, 1977, **42,** 566-8) d'après la réaction :

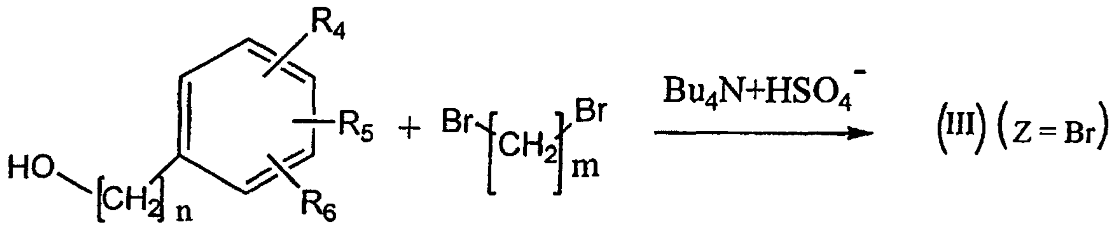


(IV) (V)

* Quand **Y** représente un triméthylène, la réaction précédente appliquée au 1,3-dibromopropane donne le dérivé attendu **III** avec un faible rendement (10 à 30 %) et il se forme surtout de l'éther allylique. Dans ce cas on préfère utiliser le 1-bromo-3- chloropropane pour préparer le dérivé chloré **III** (Z = Cl) avec un rendement de l'ordre de 50 à 60 %.
* Lorsque **Y** représente un éthylène, la réaction précédente ne peut être réalisée, il est préférable de condenser l'alcool de départ **IV** sur l'éthylène carbonate en présence d' hydrure de sodium selon **Van den Brock L.A. G., Vermaas D.J.** et coll. (*Recueil Trav. Chim. Pays-Bas*, 1994, **113,** 507-516). On obtient dans ce cas un (2-arylalcoxy) éthanol intermédiaire **VI** qui peut être activé sous forme de mésylate **III** (Z = $MeSO_3$-) après traitement par le chlorure de mésyle selon la réaction :

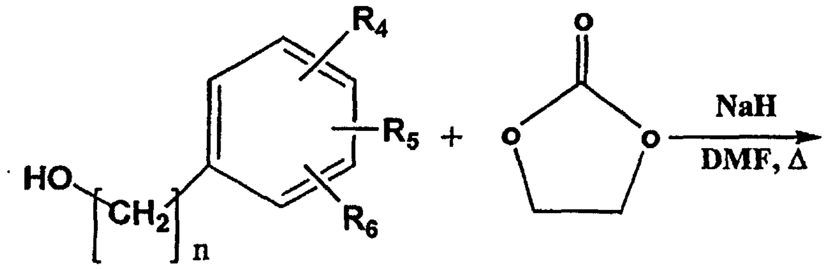


(IV)

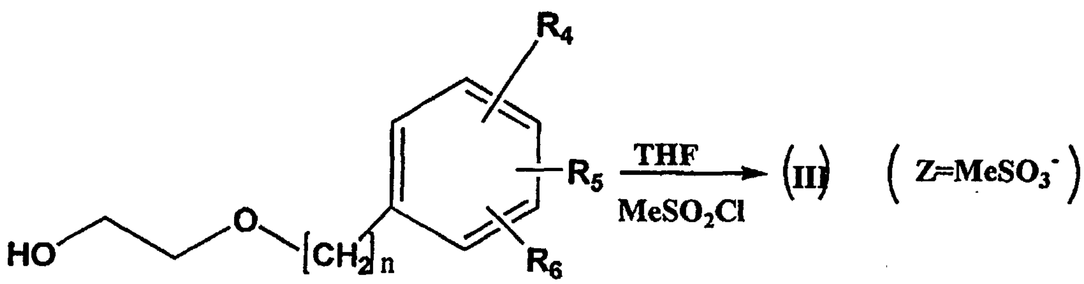


(VI)

**b)** Dans le cas où **Y représente le radical -$CH_2$-CH(OH)-$CH_2$-** les composés **I** de la présente invention sont préparés de même mais en utilisant un arylacoyloxyméthoxy époxyde **VII** comme agent alkylant selon la réaction :

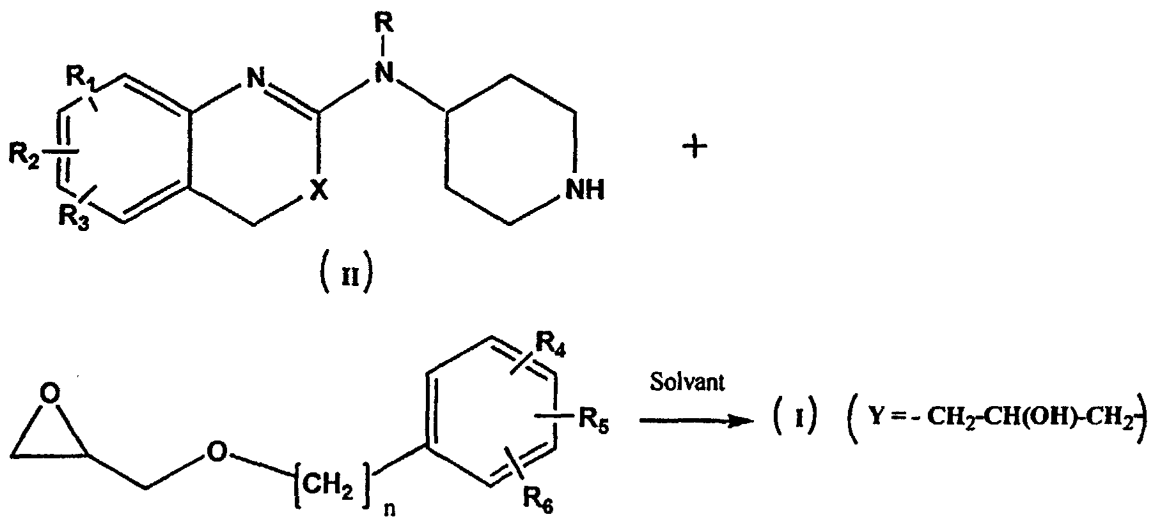


(II)

(VII)

Les éthers glycidiques **VII** sont préparés par condensation de l'épichlorhydrine sur les alcools correspondants **IV** par catalyse par transfert de phase par le procédé déjà décrit (**Mouzin G.** et coll. *Synthesis,* 1983, 117-119) selon la réaction :

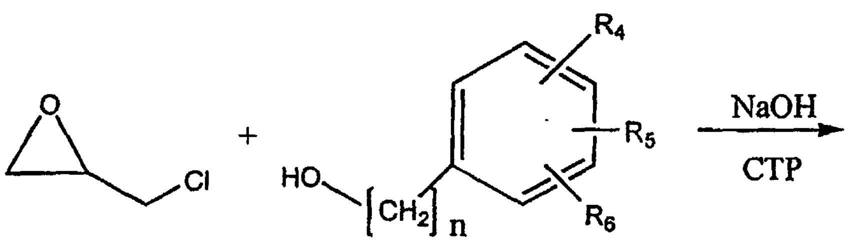


(IV)

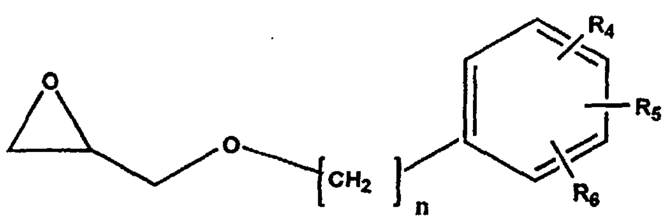

(VII)

- Lorsque les alcools de départ **IV** ne sont pas commerciaux, ils sont obtenus à partir des esters éthyliques ou méthyliques **VIII** correspondants par réduction en présence d'aluminohydrure :

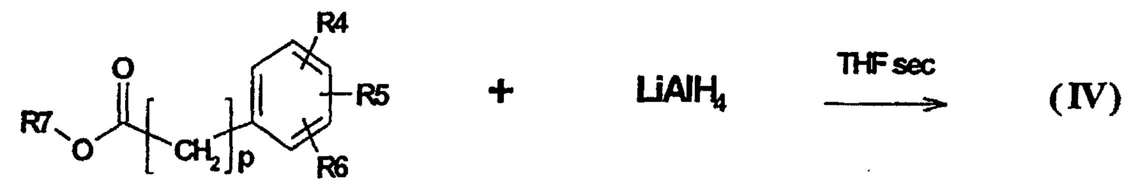


(VIII) ($R_7$=Me,Et , p=n-1)

- La synthèse de l'hétérocycle **III** a déjà été décrite dans l'application de la série de base WO-97.05314.

**2°) Préparation par hétérocyclisation finale :**

Les composés **I** peuvent être préparés par cyclisation finale en utilisant deux types d'activation des amines liées à l'hétérocycle.

a) On peut dans un premier cas **activer l'amine aromatique** sous forme **d'o-bromométhyl phényl isothiocyanate IX** comme décrit dans le brevet WO-97.05314 par réaction avec **l'aminopipéridine X** convenablement sustituée selon :

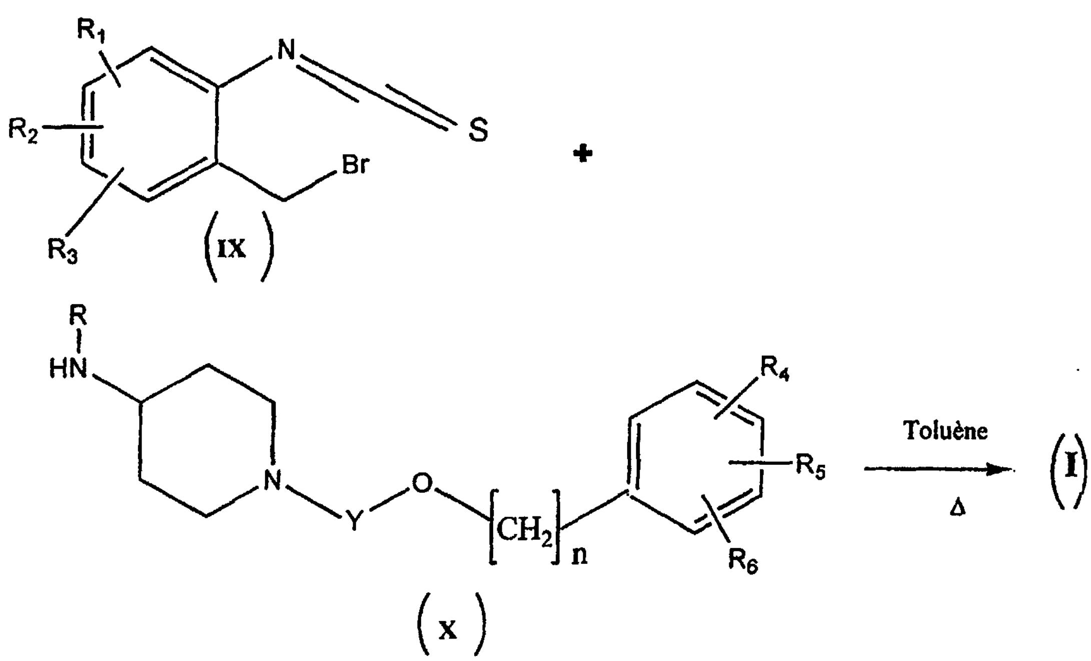


**b)L'activation** peut aussi provenir de **l'amine** portée par le noyau **pipéridinique.** Celle-ci peut être réalisée de deux manières selon la **nature** du radial **R** et se fait toujours en deux étapes :

- Si **R** est un hydrogène l'amine **X** peut être activée sous forme d'iso(thio)cyanate **XI** par réaction avec le triphosgène ou le (thio)carbonyl diimidazole selon **Staab H.A.** et **G. Walther** (*Ann.*, 1962, **657,** 104-107) par exemple :

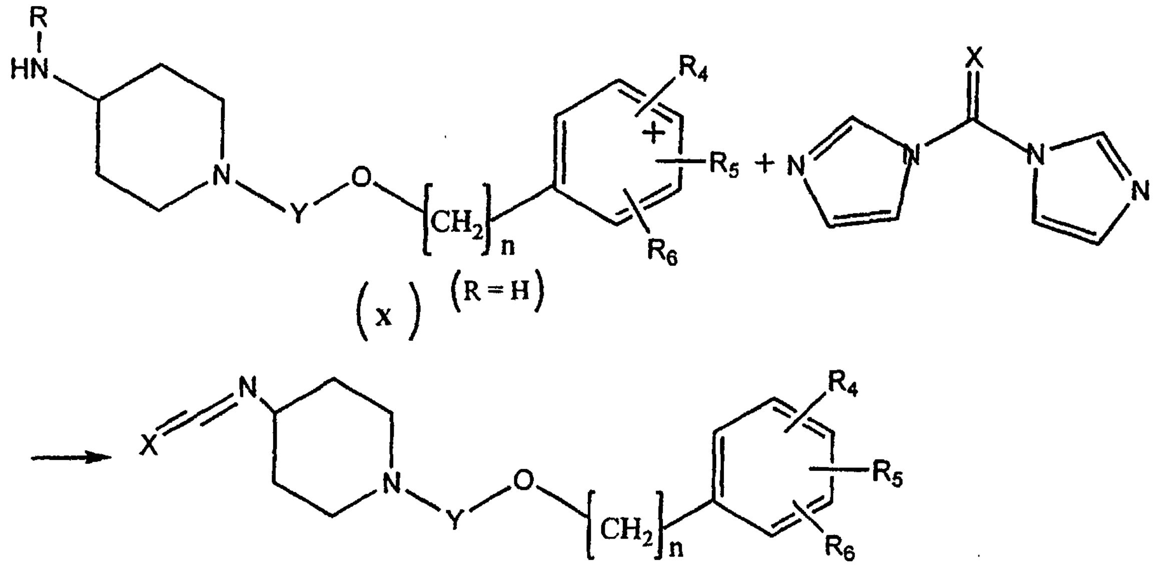

(X) (R=H)

(XI)

Ce composé réagit facilement avec un alcool orthoaminobenzylique **XII** convena-blement substitué pour donner une hydroxy(thio)urée **XIII** (R = H) qui est facilement cyclisée en milieu aqueux acide fort en composé de formule **I** objet de la présente invention comme décrit dans l'application WO-97.05314 :

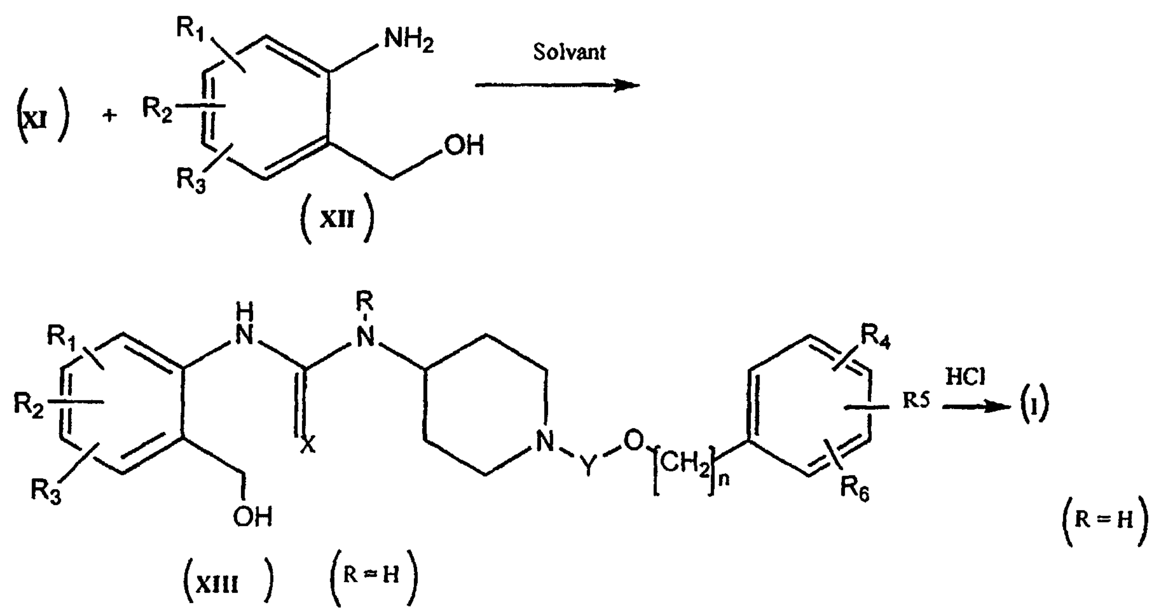


(XI) + (XII)

(XIII) (R=H) (I) (R=H)

Par N-alcoylation de **I** (**R**=H) avec un halogénure d'alcoyle après sodation avec NaH on prépare de même le composé **I** où **R** = alcoyle.

- Lorsque **R est différent d'un hydrogène** l'activation de l'amine **X** se fait avec le thiophosgène où le triphosgène pour donner un chlorure de (thio)carbamoyle **XIV** dans un solvant aprotique en milieu basique.

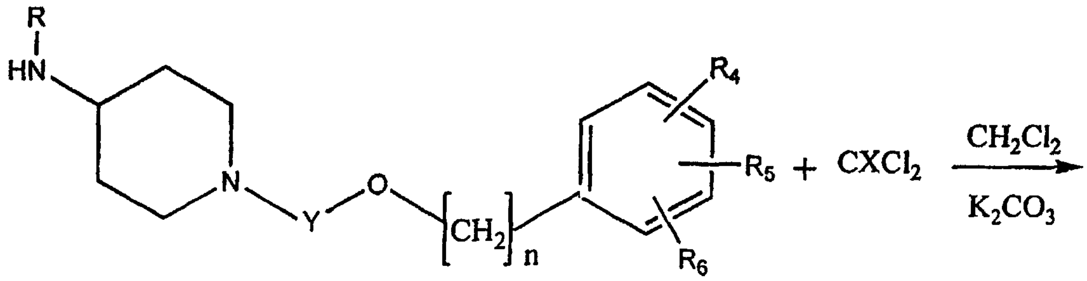


(X)

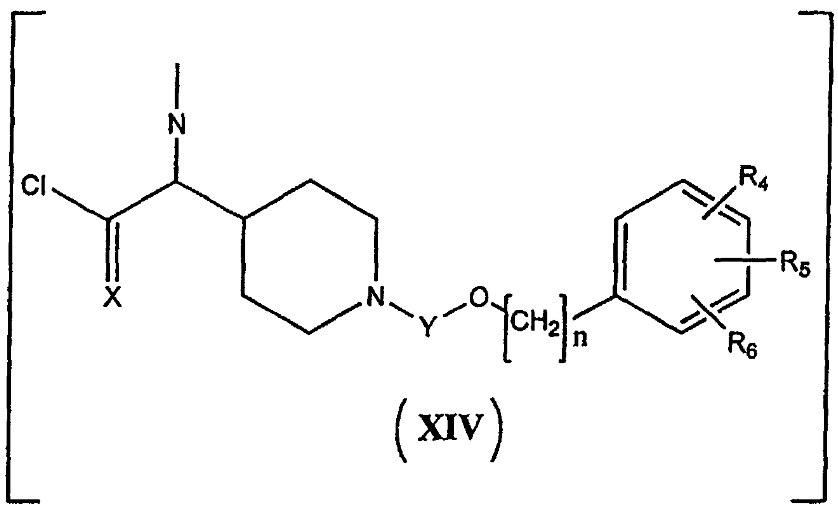

(XIV)

Ce dérivé n'est pas isolé et est ajouté directement à l'alcool o-aminobenzylique **XII** pour donner l'hydroxy(thio) urée **XIII** correspondante qui est cyclisée par le même procédé que ci-dessus :

* Lorsqu'ils ne sont pas commerciaux, les alcools amino benzyliques **XII** sont préparés par réduction des esters anthraniliques correspondants convenablement substituées.
* Les amino pipéridines **X** sont préparées en adaptant le procédé décrit dans le brevet de base WO-97.05314 par N-alcoylation des 4-pipéridones protégées **XV** avec les halogéno éthers correspondants **III,** suivie d'une déprotection du composé obtenu, pour donner la céto amine **XVI** qui subit une amination réductrice avec l'amine R-$NH_2$ (**XVII**) en présence de borane pyridine ou de triacétate de borohydrure de sodium selon le schéma :

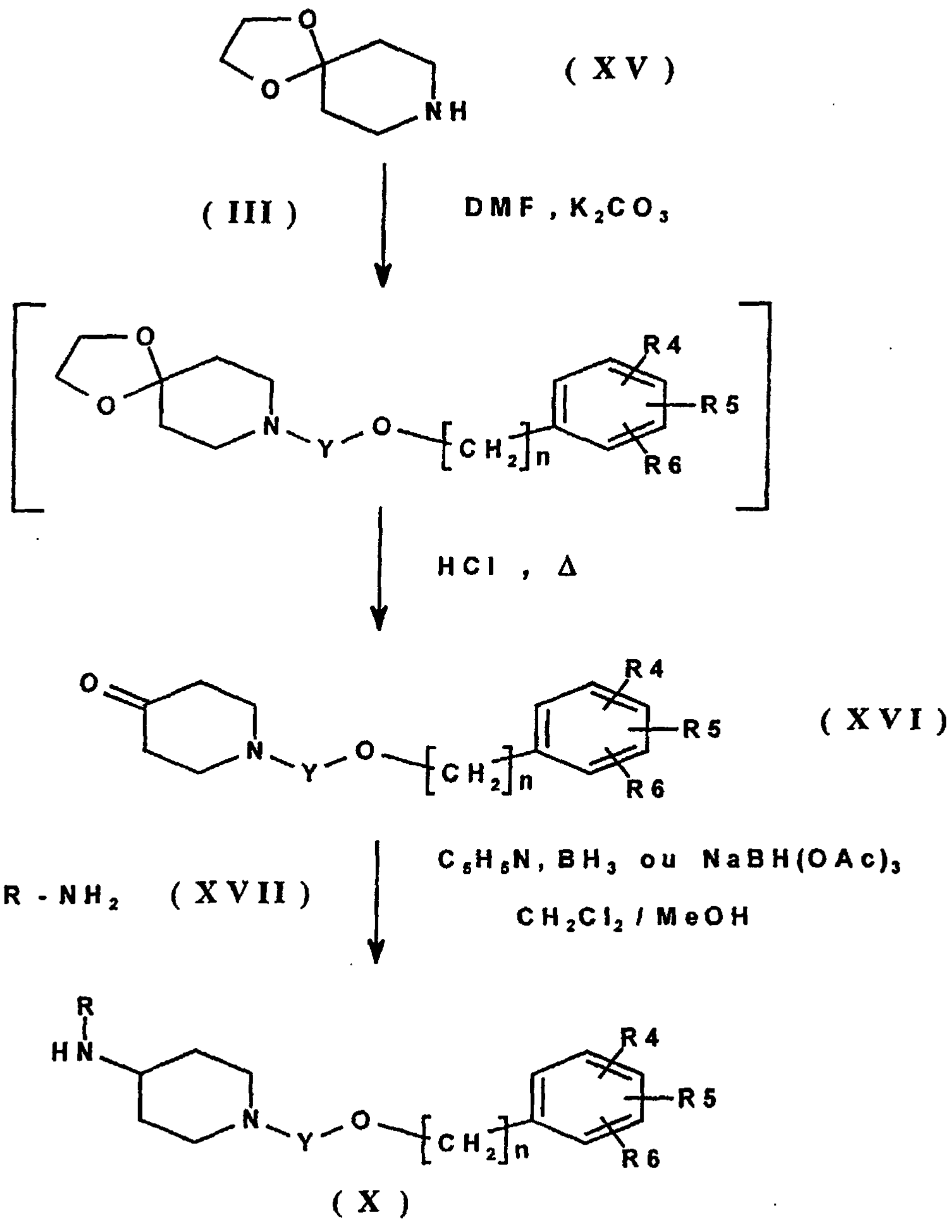


**Exemple 1 :**

**Hydrogénofumarate de la N-méthyl-N-[1-[5-(4-fluorobenzyloxy)pentyl]pipéridin -4-yl]-4H-3,1-benzothiazin-2-amine (1).**

**1.1**) 1- [5-bromopentyloxyméthyl]-4-fluorobenzène (**1.1**). Un mélange renfermant 2 g (15,8 mmol) d'alcool 4-fluorobenzylique, 18,3 g (79 mmol) de 1,5-dibromopentane, de 3,2 g (79 mmol) de soude en pastilles dans 6,4 ml d'eau et 270 mg (0,79 mmol) d'hydrogénosulfate de tetrabutyl ammonium est agité fortement pendant 2 jours à 25 °C. Le mélange réactionnel est extrait à l'éther lavé à l'eau, à l'eau salée puis séché sur sulfate de sodium anhydre. Après élimination du sel minéral, le mélange est évaporé à siccité sous vide et l'huile résiduelle est rectifiée sous vide. On récupère 5,12 g (Rdt : 63 %) d'une huile incolore de **formule 1.1 :**

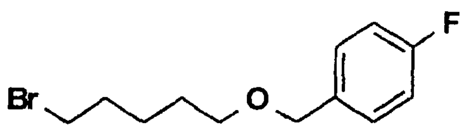

( 1-1 )

Formule brute : $C_{12}H_{16}BrFO$
Masse molaire : 275,168
Huile incolore
Point d'ébullition : 97 - 102 °C /0,1 mbar
**RMN** ($CDCl_3$) $\delta$ : 1,15- 1,73 (m, 4H) ; 1,75-1,95 (m, 2H) ; 3,28-3,56 (m, 4H) ; 4,44 (s, 2H) ; 6,9-7,1 (m, 2H) ; 7,2-7,37 (m, 2H).

**1.2)** Hydrogénofumarate de la N-méthyl-N-[1-[5-(4-fluorobenzyloxy)pentyl)pipéridin -4-yl]-4H-3,1-benzo- thiazin-2-amine (**1**). Une solution de 600 mg (2,3 mmol) de N-méthyl-N-(4-pipéridinyl)-4H-3,1-benzothiazin-2-amine (préparée selon le brevet WO-97.05134) et de 633 mg (2,3 mmol) de 1-[5-bromopentyloxyméthyl]-4-fluorobenzène dans 10 ml de DMF est traitée par 480 mg (3,5 mmol) de $K_2CO_3$ sec, broyé et un peu d'iodure de potassium, puis chauffée 3 h à 90 °C sous agitation. Après retour à 30-40 °C, le mélange est évaporé à siccité sous vide et le résidu est distribué dans un mélange $CH_2Cl_2$/eau. Les phases sont séparées. La phase organique est lavée à l'eau, à l'eau salée puis séchée sur sulfate de sodium anhydre. Le résidu obtenu après élimination des minéraux et évaporation du filtrat est purifié par flash chromatographie en éluant avec un mélange $CH_2Cl_2/CH_3OH/NH_4OH$ - 95/4,5/0,5. Les fractions renfermant le composé attendu sont réunies, évaporées à siccité pour donner 710 mg de base qui est reprise dans l'éthanol et traitée avec un équivalent d'acide fumarique dissout dans 2 ml d'éthanol chaud. On laisse revenir lentement à 25 °C puis filtre les cristaux (m = 740 mg, Rdt : 56 %) de sel de formule **1** :

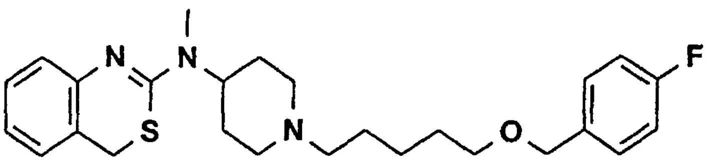 , 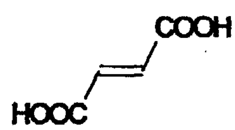

( 1 )

Formule brute : $C_{30}H_{38}FN_3O_5S$
Masse molaire : 571,71
Cristaux blancs
Point de fusion : 167 °C
**RMN** (DMSO $d_6$) $\delta$ : 1,2- 1,75 (m, 8H) ; 1,8-2,1 (m, 2H) ; 2,25-2,65 (m, 4H) ; 3 (s, 3H) ; 3,1-3,3 (m, 2H) ; 3,41 (t, 2H) ; 3,94 (s, 2H) ; 4,25-4,6 (m, 1H) ; 4,41 (s, 2H) ; 6,55 (s, 2H) ; 6,93 (t, 2H) ; 7,06-7,23 (m, 4H) ; 7,26-7,42 (m, 2H) ; 10-12 (m, 2H).

**Exemple 2 :**

**Hydrogénofumarate de la N-méthyl-N-[1-[4-(4-fluorobenzyloxy)butyl]pipéridin-4-yl]-4H-3,1-benzothiazin-2-amine (2).**

**2.1)** 1-[4-bromobutyloxyméthyl]-4-fluorobenzène (**2.1**). En appliquant le mode opératoire de l'exemple **1.1** à 10,3 g de 1,4-dibromobutane on prépare avec un rendement de 61 % le composé de **formule 2.1 :**

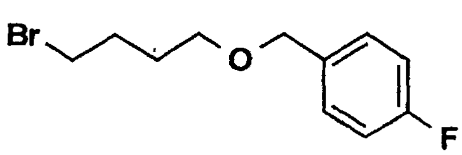

( 2-1 )

Formule brute : $C_{11}H_{14}BrFO$
Masse molaire : 261,13
Huile incolore
Point d'ébullition : 123 - 125 °C/ 0,46 mbar
**RMN** ($CDCl_3$) δ : 1,6-1,82(m , 2H) ; 1,85-2,1 (m, 2H) ; 3,32-3,58 (m, 2H) ; 4,44 (s, 2H) ; 6,92-7,08 (m, 2H) ; 7,22-7,35 (m, 2H).

**2.2**) Hydrogénofumarate de la N-méthyl-N-[1-[4-(4-fluorobenzyloxy)butyl]pipéridin-4-yl]-4H-3,1-benzothiazin-2-amine (**2**). La condensation de 660 mg (2,53 mmol) de composé bromé **2.1** ci-dessus sur 600 mg (2,3 mmol) de N-méthyl-N-(4-pipéridinyl)-4H-3,1-benzothiazin-2-amine donne 792 mg (Rdt : 62 %) de cristaux blancs de **formule 2.**

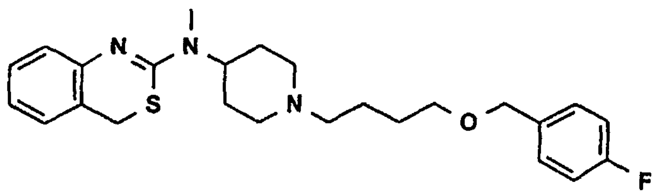 , 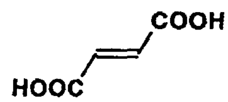

**( 2 )**

Formule brute : $C_{29}H_{36}FN_3O_5S$
Masse molaire : 557,67
Cristaux blancs
Point de fusion 174-175 °C
**RMN** ($CDCl_3$) δ : 1,4-1,7 (m, 6H) ; 1,76-2,1 (m, 2H) ; 2,32 (t, 2H) ; 2,42-2,64 ; (m, 2H) ; 2,99 (s, 3H) ; 3,06-3,15 (m, 2H) ; 3,41 (t, 2H) ; 3,91 (s, 2H) ; 4,25-4,6 (m, 1H) ; 4,4 (s, 2H) ; 6,55 (s, 2H) ; 6,91 (t, 2H) ; 7,07-7,25 (m, 4H) ; 7,25-7,4 (m, 2H) ; 10,5-12,5 (m, 2H).

**<u>Exemple 3 :</u>**

**Hydrogénofumarate de la N-méthyl-N-[1-[3-(4-fluorobenzyloxy)propyl]pipéridin-4-yl]-4H-3,1-benzothiazin-2-amine (3).**

**3.1)** 1-(3-bromopropyloxyméthyl)-4-fluorobenzène (**3.1**). La condensation de 16 g de 1,3-dibromopropane sur 2 g (15,8 mmol) d'alcool 4-fluorobenzylique selon le procédé de l'exemple **1.1** fournit après distillation 1,05 g (Rdt : 27 %) d'une huile incolore de **formule 3.1 :**

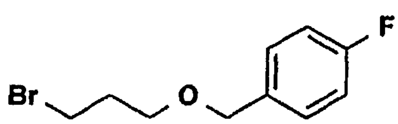

**( 3-1 )**

Formule brute : $C_{10}H_{12}BrFO$
Masse molaire : 247,11
Huile incolore
Point d'ébullition : 92-95 °C (0,63 mbar)
**RMN** ($CDCl_3$) δ : 2,05-2,25 (m, 2H) ; 3,45-3,70 (m, 4 H) ; 4,48 (s, 2H) ; 6,95-7,12 (m , 2H) . 7,22-7,4 (m, 2H).

**3.2)** Hydrogénofumarate de la N-méthyl-N-[1-[3-(4-fluorobenzyloy)propyl]pipéridin-4-yl]-4H-3,1-benzothiazin-2-amine (**3**). La N-alcoylation de 586 mg (2,24 mmol) de N-méthyl-N-(4-pipéridinyl)-4H-3,1-benzothiazin-2-amine par 582 mg (2,35 mmol) de dérivé bromé précédent **3.1** selon l'exemple **1.2** donne 785 mg (Rdt : 64 %) de composé de **formule 3 :**

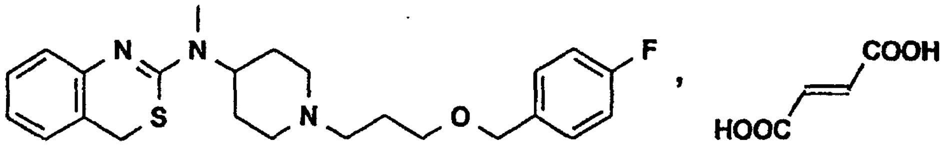

(3)

Formule brute : $C_{28}H_{34}FN_3O_5S$
Masse moléculaire : 543,64
Cristaux blancs
Point de fusion : 163-164 °C
**RMN** (DMSO $d_6$) δ : 1,57-2,1 (m, 6H) ; 2,34 (t, 2H) ; 2,55-2,7 (m, 2H) ; 3,02 (s, 3H) ; 3,08-3,2 (m, 2H) ; 3,48 (t, 2H) ; 3,96 (s, 2H) ; 4,25-4,57 (m, 1H) ; 4,45 (s, 2H) ; 6,6 (s, 2H) ; 6,95 (t, 2H) ; 7,05-7,28 (m, 4H) ; 7,3-7,5 (m, 2H) ; 11-13 (m, 2H).

**Exemple 4 :**

**Hydrogénofumarate de la N-méthyl-N-[1-[2-(4-fluorobenzyloxy)éthyl]pipéridin-4-yl]-4H-3,1-benzothiazin-2-amine (4).**

**4.1.)** 4-fluoro-1-[2-hydroxyéthoyméthyl]benzène (**4.1**). Une solution de 4 g (31,7 mmol) d'alcool 4-fluorobenzylique dans 30 ml de DMF sec est refroidie à 0 °C puis traitée en 25 min par 3,8 g (95 mmol) d'hydrure de sodium à 60 %. L'agitation est ensuite poursuivie 100 min à 25 °C puis on ajoute, goutte à goutte, 3,6 g (63,4 mmol) de carbonate d'éthylène en solution dans 20 ml de DMF et on agite 1h à 25 °C. Le mélange réactionnel est versé sur de la glace pilée, extrait à l'éther lavé à l'eau, à l'eau salée, et séché sur sulfate de sodium anhydre. Le sel minéral est filtré et le filtrat est évaporé à siccité, puis l'huile résiduelle est purifiée par flash chromatographie en éluant avec un mélange éther de pétrole/acétate d'éthyle 90/10 puis 70/30. Les fractions renfermant l'éther attendu sont récupérées de la manière habituelle pour donner 5,95 g (Rdt : 56 %) d'hydroxy éther de **formule 4.1 :**

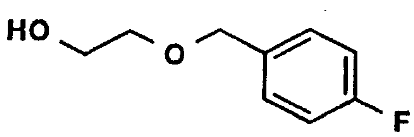

(4-1)

Formule brute : $C_9H_{11}FO_2$
Masse molaire : 170,18
Huile jaune clair
**RMN** ($CDCl_3$) δ : 2,1 (m, 1H) ; 3,52-3,63 (m, 2H) ; 3,75 (t, 2H) ; 4,51 (s, 2H) ; 6,95-7,12 (m, 2H) ; 7,14-7,45 (m, 2H).

**4.2)** Mésylate de 2-[4-fluorobenzyloxy]éthyle (**4.2**). Une solution de 1,5 g (8,8 mmol) d'alcool précédent **4.1** dans 15 ml de THF sec est refroidie à 0 °C, puis traitée avec 1,84 ml (1,34 g ; 13,2 mmol) de triéthylamine et goutte à goutte avec 1,11 g (9,7 mmol) de chlorure de mésyle. On laisse revenir à 25 °C et on maintient ½ h de plus à cette température, puis le mélange est filtré et on ajoute 20 ml d'eau au filtrat et extrait à l'éther, puis lave à l'eau, à l'eau salée, et sèche sur sulfate de sodium. Le mésylate est récupéré après évaporation à siccité m = 2,29 g (Rdt : 92 %) et a pour **formule 4.2 :**

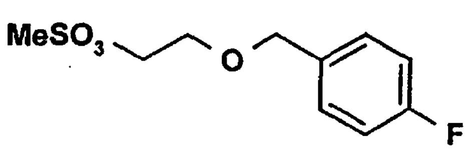

(4-2)

Formule brute : $C_{10}H_{13}FO_4S$
Masse molaire : 248,26
Huile jaune
**RMN** ($CDCl_3$) δ : 3,03 (s, 3H) ; 3,65-3,8 (m, 2H) ; 4,35-4,45 (m, 2H) ; 4,54 (s, 2H) ; 6,95-7,12 (m, 2H) ; 7,22-7,38 (m, 2H).

**4.3)** Hydrogénofumarate de la N-méthyl-N-[1-[2-(4-fluorobenzyloxy)éthyl]pipéridin-4-yl]-4H-3,1-benzothiazine-2-amine (**4**). En utilisant le mésylate de 2-[4-fluoroben- zyloxy]éthyle (627 mg ou 2,53 mmol) et en appliquant le procédé de l'exemple **1.2** on prépare 920 mg (Rdt : 76 %) de poudre blanche de **formule 4 :**

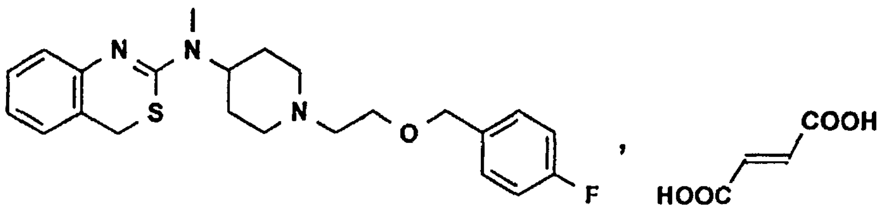

**(4)**

Formule brute : $C_{27}H_{32}FN_3O_5S$
Masse molaire : 529,61
Cristaux blancs
Point de fusion : 160 - 161 °C
**RMN** (DMSO $d_6$) δ : 1,45-2,05 (m, 4H) ; 2,33 (t, 2H) ; 2,71 (t, 2H) ; 3,01 (s, 3H) ; 3-3,15 (m, 2H) ; 3,59 (t, 2H) ; 3,93 (s, 2H) ; 4,25-4,52 (m, 1H) ; 4,47 (s, 2H) ; 6,59 (s, 2H) ; 6,93 (t, 2H) ; 7,05-7,28 (m, 4H) ; 7,3-7,46 (m, 2H) ; 11-3 (m, 2H).

**<u>Exemple 5 :</u>**

**Hydrogénofumarate de la N-méthyl-N-[1-[4-[2-(4-fluorophényl)éthoxy]butyl] pipéridin-4-yl]-4H-3,1-benzothiazin-2-amine (5).**

**5.1)** 4-fluoro-1-[2-[4-bromobutyloxy]éthyl]benzène (**5.1**). La O-alcoylation de 2 g (14.3 mmol) d'alcool 4-fluorophénéthylique par 15,4 g (71,3 mmol) de 1,4-dibromobutane selon **1.1** donne 3,1 g (Rdt : 79 %) de composé de **formula 5.1 :**

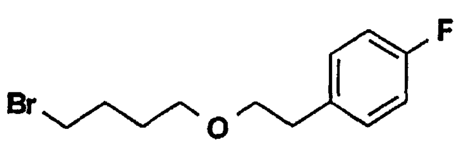

**(5-1)**

Formule brute : $C_{12}H_{16}BrFO$
Masse molaire : 275,16
Huile incolore
Point d'ébullition : 115 - 120 °C / 0,24 mbar
**RMN** ($CDCl_3$) δ : 1,6-1,8 (m, 2H) ; 1,8-2,05 (m, 2H) ; 2,85 (t, 2H) ; 3,3-3,5 (m, 4H) ; 3,6 (t, 2H) ; 6,9-7,05 (m, 2H) ; 7,07-7,26 (m, 2H).

**5.2)** Hydrogénofumarate de la N-méthyl-N-[1-[4-[2-(4-fluorophényl)éthoxy]butyl] pipéridin-4-yl]-4H-3,1-benzothiazin-2-amine (**5**). L'application du procédé **1.2** à 665 mg (2,41 mmol) de dérivé bromé **5.1** précédent permet de préparer 780 mg (Rdt : 59%) de sel de **formule 5 :**

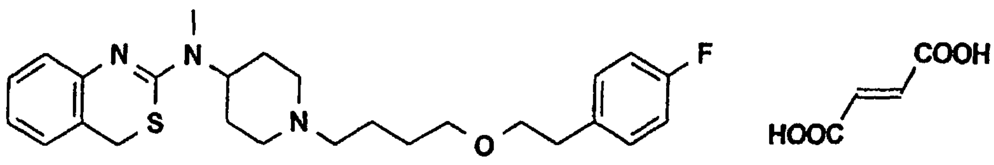

(5)

Formule brute : $C_{30}H_{38}FN_3O_5S$
Masse molaire : 571,69
Cristaux blancs
Point de fusion : 173-174 °C
**RMN** (DMSO $d_6$) δ : 1,35-2,1 (m, 8H) ; 2,29 (t, 2H) ; 2,42-2,57 (m, 2H) ; 2,78 (t, 2H) ; 3 (s, 3H) ; 3-3,2 (m, 2H) ; 3,37 (t, 2H) ; 3,53 (t, 2H) ; 3,93 (s, 2H) ; 4,39 (m, 1H) ; 6,56 (s, 2H) ; 6,92 (t, 2H) ; 7-7,32 (m, 6H) ; 11,5-13 (m, 2H).

**Exemple 6 :**

**Hydrogénofumarate de la N-méthyl-N-[1-[3-[2-(4-fluorophényl)éthoxy]propyl] pipéridin-4-yl]-4H-3,1-benzothiazin-2-amine (6).**

**6.1)** 4-fluoro-1-[2-(3-chloropropoxy)éthyl]benzène **6.1.** Une solution de 86 g (2,14 mol) de NaOH en pastilles dans 86 g d'eau est agitée puis refroidie à 25 °C puis traitée ave 20 g (0,143 mol) d'alcool 4-fluorophénéthylique, avec 113 ml (1,14 mol) de 1-bromo-3-chloropropane puis avec 4,85 g (14 mmol) d'hydrogénosulfate de tétrabutyl ammonium. Une forte agitation est maintenue pendant 4 h à 25 °C, puis on extrait à l'éther, lave à l'eau, à l'eau salée et sèche sur sulfate de sodium anhydre. Après élimination du sel minéral, le filtrat est évaporé à siccité et l'huile résiduelle est rectifiée sous vide pour donner 20,3 g (Rdt : 65 %) de produit de **formule 6.1 :**

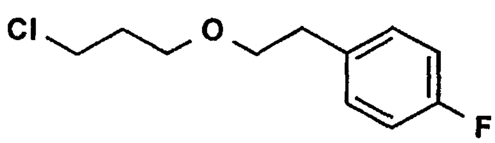

(6-1)

Formule brute : $C_{11}H_{14}ClFO$
Masse molaire : 216,67
Huile incolore
Point d'ébullition : 94-97 °C / 0,4 mbar
**RMN** ($CDCl_3$) δ : 2,08 (q, 2H) ; 2,85 (t, 2H) ; 3,46 (t, 2H) ; 3,55 (t, 2H) ; 3,63 (t, 2H) ; 6,97 (t, 2H) ; 7,1-7,2 (m, 2H).

**6.2)** Hydrogénofumarate de la N-méthyl-N-[1-[3-[2-(4-fluorophényl)éthoxy]propyl] pipéridin-4-yl]-4H-3,1-benzothiazin-2-amine (**6**). Une solution de 1,48 g (6,83 mmol) de composé précédent **6.1** et de 1,7 g (6,5 mmol) de N-méthyl-N-[4-pipéridinyl]-4H-3,1-benzothiazin-2-amine dans 18 ml de DMF, est traitée avec 945 mg (6,83 mmol) de $K_2CO_3$ broyé et 20 mg de KI puis, portée pendant 4 h sur bain d'huile à 80 °C. Après retour à 25 °C le mélange est traité comme dans l'exemple **1.2** pour donner après purification par flash chromatographie 3,12 g de base brute, qui est salifiée de la manière habituelle pour donner le composé de **formule 6.2** (Rdt : 68 %).

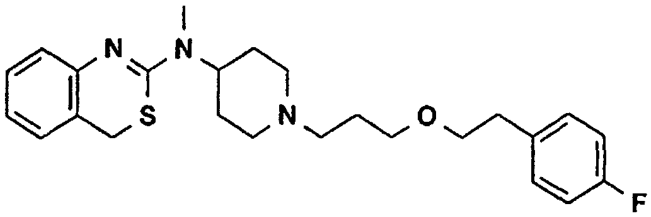, 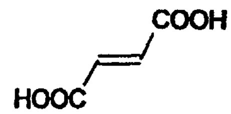 **(6)**

Formule brute : $C_{29}H_{36}FN_3O_5S$
Masse molaire : 557,67
Cristaux blancs
Point de fusion : 163-4 °C
**RMN** (DMSO $d_6$) $\delta$ : 1,6-1,72 (m, 4H) ; 1,75-1,95 (m, 2H) ; 2,24 (t,2H) ; 2,4-2,58 (m, 2H) ; 2,79 (t, 2H) ; 3,02 (s, 3H) ; 3-3,14 (m, 2H) ; 3,41 (t, 2H) ; 3,55 (t, 2H) ;3,94 (s, 2H) ; 4,37 (m, 1H) ; 6,58 (s, 2H) ; 6,85-6,98 (m, 2H) ; 7,02-7,2 (m, 4H) ; 7,25-7,3 (m, 2H) ; 10-12 (m, 2H).

**Exemple 7 :**

**Dichlorhydrate de la N-méthyl-N-[1-[3-[2-(4-fluorophényl)éthoxy]propyl]pipéridin-4-yl]-4H-3,1-benzothiazin-2-amine (7).**

Une solution de 425 mg de base obtenue dans l'exemple **6.2** dans 4 ml d'éthanol sec est refroidie sur bain de glace, puis traitée goutte à goutte avec 0,9 ml d'une solution 2,3 N d'acide chlorhydrique dans l'éthanol. Après 10 minutes d'agitation, on ajoute 3 à 4 volumes d'éther éthylique en amorçant avec une baguette de verre. On abandonne une nuit au réfrigérateur puis filtre les cristaux de sel formé, qui sont rinçés à l'éther puis séchés dans une cloche à vide en présence d'actigel : m = 512 mg (Rdt : 63 %) de cristaux de **formule 7 :**

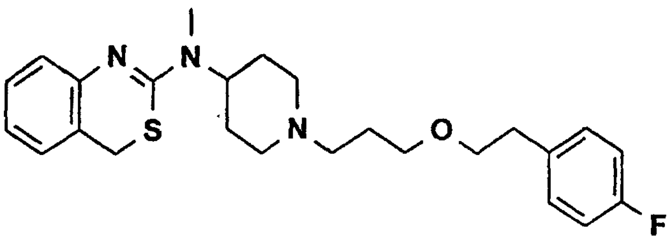 , **2 HCl** **(7)**

Formule brute : $C_{25}H_{34}Cl_2FN_3OS$
Masse molaire : 514,52
Cristaux blancs hygroscopiques (renfermant 3,2 % $H_2O$)
Point de décomposition > 110 °C
**RMN** (DMSO $d_6$) 1,88-2,08 (m, 4H) ; 2,4-2,53 (m, 2H) ; 2,81 (t, 2H) ; 3-3,23 (m, 4H) ; 3,27 (s, 3H) ; 3,47 (t, 2H) ; 3,5-3,65 (m, 4H) ; 4,3 (s, 2H) ; 4,57 (m, 1H) ; 7,11 (t, 2H) ; 7,2-7,45 (m, 6H) ; 7,54 (m, 1H) ; 11,16 (m, 1H).

**Exemple 8 :**

**Hydrogénofumarate de la N-méthyl-N-[1-[3-[3-(4-fluorophenyl)propoxy]propyl] pipéridin-4-yl]-4H-3,1-benzothiazin-2-amine (8).**

**8.1)** 1-[3-(3-bromopropoxy)propyl]-4-fluorobenzène (**8.1**). La réaction d'éthérifi- cation réalisée selon le protocole **1.1** à partir de 2 g (13 mmol) d'alcool 3-(4-fluorophényl)propylique en présence de 13 g de 1,3-dibromopropane, donne après purification par flash chromatographie 1,05 g (Rdt : 29 %) de composé de **formule 8.1 :**

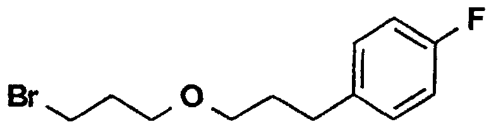

**(8-1)**

Formule brute : $C_{12}H_{16}BrFO$
Masse molaire : 275,17
Huile ambrée
**RMN** ($CDCl_3$) δ : 1,75-1,95 (m, 2H) ; 2-2,18 (m, 2H) ; 2,64 (t, 2H) ; 3,4 (t, 2H) ; 3,51 (t, 4H) ; 6,95 (t, 2H) ; 7,05-7,2 (m, 2H).

**8.2)** Hydrogénofumarate de la N-méthyl-N-[1-[3-[3-(4-fluorophényl)propoxy]propyl] pipéridin-4-yl]-4H-3,1-benzothiazin-2-amine (**8.2**). En utilisant le procédé de l'exemple **1.1** mais en partant de 600 mg (2,3 mmol) de dérivé bromé **8.1** précédent, on prépare 995 mg (Rdt : 75 %) du composé de **formule 8 :**

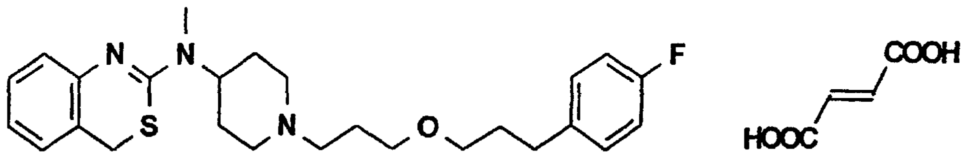

**(8)**

Formule brute : $C_{30}H_{38}FN_3O_5S$
Masse molaire : 571,71
Cristaux blancs
Point de fusion : 176 °C
**RMN** (DMSO $d_6$) δ : 1,57-2,07 (m, 8H) ; 2,34 (t, 2H) ; 2,52-2,66 (m, 4H) ; 3,01 (s, 3H) ; 3,05-3,22 (m, 2H) ; 3,25-3,45 (m, 4H) ; 3,94 (s, 2H) ; 4,04 (m, 1H) ; 6,57 (s, 2H) ; 6,93 (t, 2H) ; 7-7,3 (m, 6H) ; 10-12 (m, 2H).

**<u>Exemple 9 :</u>**

**Hydrogénofumarate de la N-méthyl-N-[1-[2-[2-(4-fluorophényl)éthoxy]éthyl] pipéridin-4-yl]-4H-3,1-benzothiazin-2-amine (9).**

**9.1)** 4-fluoro-1-[2-[2-hydroxyéthoxy]éthyl]benzène (**9.1**). En partant de 4 g (28,5 mmol) d'alcool 4-fluorophénéthylique et de 5,03 g (57 mmol) de carbonate d'éthylène selon le protocole **4.1,** on prépare 2,1 g (Rdt : 40 %) d'hydroxyéther de **formula 9.1 :**

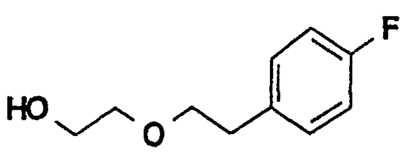

**(9-1)**

Formule brute :$C_{10}H_{13}FO_2$
Massse molaire : 184,20
Huile jaune clair
**RMN** ($CDCl_3$) δ : 1,82 (s, 1H) ; 2,86 (t, 2H) ; 3,5-3,8 (m, 6H) ; 6,9-7,05 (m, 2H) ; 7,12-7,24 (m, 2H).

**9.2)**Mésylate de 2-[2-(4-fluorophényl)éthoxy]éthyle (**9.2**). 900 mg d'alcool précédent **9.1** sont traités en présence de 616 mg (5,4 mmol) de chlorure de mésyle selon **4.2,** pour donner 1,17 g (Rdt : 91 %) d'une huile jaune de

**formule 9.2 :**

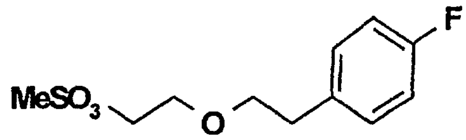

**( 9-2 )**

Formule brute : $C_{11}H_{15}FO_4S$
Masse polaire : 262,29
Huile jaune
**RMN** ($CDCl_3$) δ : 2,83 (t, 2H) ; 2,92 (s, 3H) ; 3,55-3,7 (m, 4H) ; 4,26-4,37 (m, 2H) ; 6,9-7,04 (m, 2H) ; 7,08-7,23 (m, 2H).

**9.3)** Hydrogénofumarate de la N-méthyl-N-[1-[2-[2-(4-fluorophényl)éthoxy]éthyl] pipéridin-4-yl]-4H-3,1-benzothiazin-2-amine (**9**). En opérant selon le procédé de l'exemple **1.2** à partir de 773 mg (2,95 mmol) de mésylate **9.2** précédent, on obtient 920 mg (Rdt : 63 %) de composé de **formula 9 :**

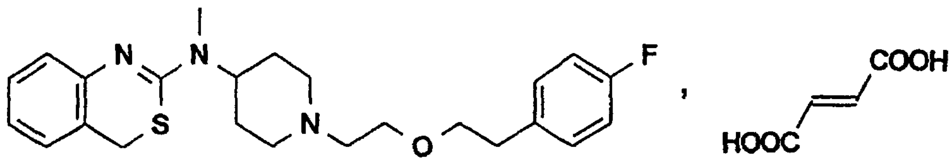

**( 9 )**

Formule brute : $C_{28}H_{34}FN_3O_5S$
Masse molaire : 543,64
Cristaux blancs pulvérulents
Point de fusion : 145-6 °C
**RMN** (DMSO $d_6$) δ : 1,52-1,68 (m, 2H) ; 1,72-1,9 (m, 2H) ; 2,23 (t, 2H) ; 2,61 (s, 2H) ; 2,8 (t, 2H) ; 2,95-3,1 (m, 5H) ; 3,5-3,63 (m, 4H) ; 3,94 (s, 2H) ; 4,32 (m, 1H) ; 6,6 (s, 2H) ; 6,88-6,97 (m, 2H) ; 7,05-7,21 (m, 4H) ; 7,25-7,32 (m, 2H) ; 11-13 (m, 2H).

**Exemple 10 :**

**Hydrogénofumarate de la N-méthyl-N-[1-[5-(3,4-difluorophénylméthoxy)pentyl] pipéridin-4-yl]-4H-3,1-benzothiazin-2-amine (10).**

**10.1)** 1-(5-bromopentyloxyméthyl)-3,4-difluorobenzène (**10.1**). L'éthérification de 2 g (13,9 mmoles) d'alcool 3,4-difluorobenzylique en présence de 16 g de 1,5-dibromopentane, selon le procédé décrit dans l'exemple **1.1** fournit 2,96 g (Rdt : 72 %) de composé de **formule 10.1 :**

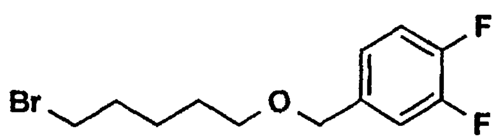

**( 10-1 )**

Formule brute : $C_{12}H_{15}BrF_2O$
Masse molaire : 293,16
Huile incolore
Point d'ébullition : 110-120 °C / 0,1 mbar

**RMN** ($CDCl_3$) δ : 1,4-1,74 (m, 4H) ; 1,77-1,98 (m, 2H) ; 3,33-3,56 (m, 4H) ; 4,44 (s, 2H) ; 6,95-7,24 (m, 3H).

**10.2)** Hydrogénofumarate de la N-méthyl-N-[1-[5-(3,4-difluorophénylméthoxy) pentyl]pipéridin-4-yl]-4H-3,1-benzothiazin-2-amine (**10**). En partant de 674 mg (2,3 mmol) de dérivé bromé précédent **10.1** et en les condensant sur 600 mg (2,3 mmol) de N-méthyl-N-(4-pipéridinyl)-4H-3,1-benzothiazin-2-amine selon **1.2,** on prépare 882 mg de cristaux (Rdt : 65 %) de **formula 10 :**

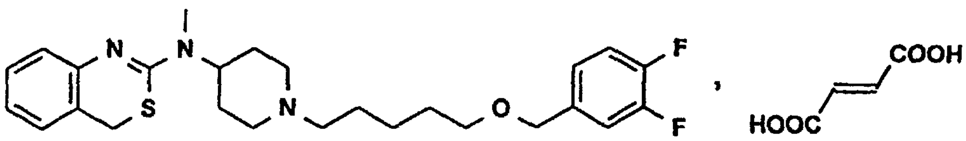

**( 10 )**

Formule brute : $C_{30}H_{37}F_2N_3O_5S$
Masse moléculaire : 589,71
Cristaux blanc cassé
Point de fusion : 169 °C
**RMN** (DMSO $d_6$) δ : 1,2-1,8 (m, 8H) ; 1,8-2,07 (m, 2H) ; 2,3 (t, 2H) ; 2,4-2,6 (m, 2H) ; 3,01 (s, 3H) ; 3-3,25 (m, 2H) ; 3,43 (t, 2H) ; 3,95 (s, 2H) 4,25-4,55 (m, 1H) ; 4,44 (s, 2H) ; 6,58 (s, 2H) ; 6,96 (t, 2H) ; 7,03-7,27 (m, 3H) ; 7,3-7,45 (m, 2H) ; 11-13 (m, 2H).

**Exemple 11 :**

**Hydrogénofumarate de la N-méthyl-N-[1-[4-(3,4-difluorophénylméthoxy)butyl] pipéridin-4-yl]-4H-3,1-benzothiazin-2-amine (11).**

**11.1)**1-(4-bromobutoxyméthyl)-3,4-difluorobenzène (**11.1**). L'application du proto- cole **1.1** à 2 g (13,9 mmol) d'alcool 3,4-difluorobenzylique et à 15 g de 1,4-dibromobutane donne 2,80 g (Rdt : 72 %) de composé de **formule 11.1 :**

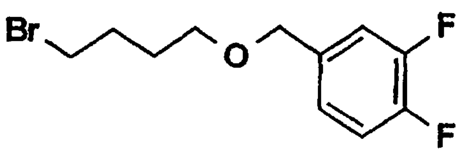

**( 11-1 )**

Formule brute : $C_{11}H_{13}BrF_2O$
Masse molaire : 279,13
Huile incolore
Point d'ébullition : 120-125 °C / 0,3 mbar
**RMN** ($CDCl_3$) δ : 1,65-1,85(m, 2H) ; 1,89-2,07 (m, 2H) ; 3,38-3,58 (m, 4H) ; 4,44 (s, 2H) ; 6,95-7,25 (m, 3H).

**11.2)** Hydrogénofumarate de la N-méthyl-N-[1-[4-(3,4-difluorophénylméthoxy)butyl] pipéridin-4-yl]-4H-3,1-benzothiazin-2-amine (**11**). L'application du procédé **1.1** à 642 mg (2,3 mmol) de dérivé bromé précédent **11.1** permet de préparer 790 mg (Rdt : 60 %) du dérivé de **formule 11 :**

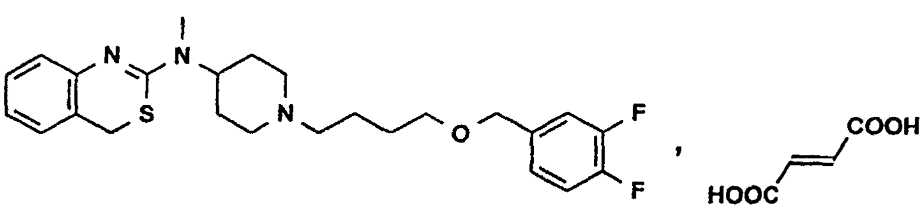

**( 11 )**

Formule brute : $C_{29}H_{35}F_2N_3O_5S$
Masse molaire : 575,68
Cristaux blancs
Point de fusion : 170 °C
**RMN** (DMSO $d_6$) δ : 1,45-1,76 (m, 6H) ; 1,78-2,08 (m, 2H) ; 2,3 (t, 2H) ; 2,4-2,6 (m, 2H) ; 3,02 (s, 3H) ; 3-3,22 (m, 2H) ; 3,45 (t, 2H) ; 3,95 (s, 2H) ; 4,25-4,55 (m, 1H) ; 4,45 (s, 2H) ; 6,58 (s, 2H) ; 6,94 (t, 2H) ; 7,1-7,25 (m, 3H) ; 7,3-7,45 (m, 2H) ; 10,5-12,5 (m, 2H).

**Exemple 12 :**

**Hydrogénofumarate de la N-méthyl-N-[1-[3-(3,4-difluorophénylméthoxy)propyl] pipéridin-4-yl]-4H-3,1-benzothiazin-2-aminé (12).**

**12.1)** 1-(3-bromopropyloxyméthyl)-3,4-difluorobenzène (**12.1**). Ce composé a été préparé selon le protocole **1.1** avec un rendement de 21 % et a pour **formula 12.1 :**

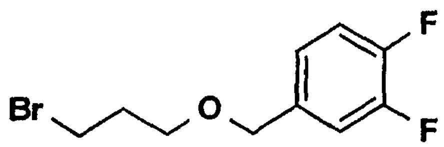

**( 12-1 )**

Formule brute : $C_{10}H_{11}BrF_2O$
Masse molaire : 265,10
Huile incolore
Point d'ébullition :92-94 °C / 0,18 mbar
**RMN** ($CDCl_3$) δ : 2,13 (q, 2H) ; 3,45-3,65 (m, 4H) ; 4,46 (s, 2H) ; 6,95-7,23 (m, 3H).

**12.2)** Hydrogénofumarate de la N-méthyl-N-[1-[3-(3,4-difluorophénylméthoxy)pro-pyl]pipéridin-4-yl]-4H-3,1-benzothiazin-2-amine (**12**). La condensation de 330 mg (1,25 mmol) de composé précédent **12.1** selon le protocole **1.2**, donne 370 mg (Rdt : 52 %) de poudre de **formule 12** :

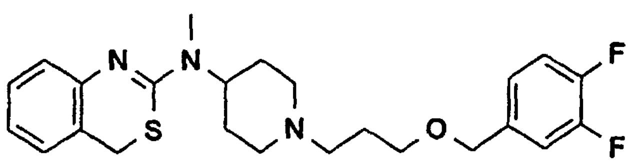

,

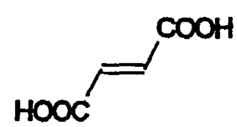

**( 12 )**

Formule brute : $C_{28}H_{33}F_2N_3O_5S$
Masse molaire : 561,65
Cristaux blancs pulvérulents
Point de fusion : 168 °C
**RMN** (DMSO $d_6$) δ : 1,53-2,06 (m, 6H) ; 2,3 (t, 2H) ; 2,5-2,68 (m, 2H) ; 3,01 (s, 3H) ; 3-3,22 (m, 2H) ; 3,48 (t, 2H) ; 3,94 (s, 2H) ; 4,28-4,52 (m, 1H) ; 4,44 (s, 2H) ; 6,58 (s, 2H) ; 6,93 (t, 2H) ; 7,1-7,27 (m, 3H) ; 7,3-7,45 (m, 2H) ; 10,5-12,5 (m, 2H).

**Exemple 13 :**

**Hydrogénofumarate de la N-méthyl-N-[1-[4-[2-(3,4-difluorophényl)éthoxy]butyl] pipéridin-4-yl]-4H-3,1-benzothiazin-2-amine (13).**

**13.1)**Alcool 3,4-difluorophénéthylique (**13.1**). Une solution de 5 g (29 mmol) de 3,4-difluorophénylacétate de méthyle (préparé par réaction de Fischer à partir de l'acide correspondant avec un rendement de 92 %) dans 30 ml de THF anhydre, est traitée par 29 ml d'une solution 2 M de $LiAlH_4$ dans le THF à 0 °C. Après agitation pendant une heure de plus, on ajoute goutte à goutte 30 ml d'acétate d'éthyle puis, 10 ml d'eau puis on agite 10 min à 25 °C, sèche sur sulfate de sodium anhydre et élimine l'insoluble minéral. Le filtrat est évaporé à siccité et purifié par flash chromatographie, en éluant avec un mélange éther de pétrole / acétate d'éthyle 97/03. On obtient ainsi 3,6 g (Rdt : 77%) d'alcool de **formule 13.1 :**

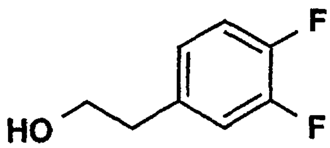

**( 13-1 )**

Formule brute : $C_8H_8F_2O$
Masse molaire : 158,15
Huile légèrement ambrée
**RMN** ($CDCl_3$) δ : 2,39 (s, 1H) ; 2,77 (t, 2H) ; 3,77 (t, 2H) ; 6,82-7,15 (m, 3H).

**13.2)** 1-[2-(4-bromobutoxy)éthyl]-3,4-difluorobenzène **13.2.** En partant de 1,2 g (7,6 mmoles) d'alcool précédent **13.1** par réaction selon le procédé **1.1** on prépare avec un rendement de 78 % le composé de **formule 13.2** :

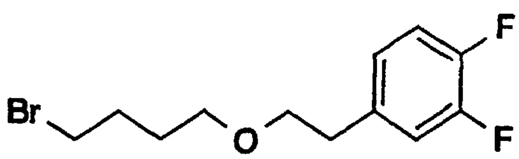

**( 13-2 )**

Formule brute : $C_{12}H_{15}BrF_2O$
Masse molaire : 293,16
Huile légèrement ambrée
**RMN** ($CDCl_3$) δ : 1,6-1,78 (m, 2H) ; 1,81-2,04 (m, 2H) ; 2,83 (t, 2H) ; 3,36-3,52 (m, 4H) ; 3,61 (t, 2H) ; 6,85-7,2 (m, 3H).

**13.3)** Hydrogénofumarate de la N-méthyl-N-[1-[4- [2- (3,4-difluorophényl)éthoxy] butyl]pipéridin-4-yl]-4H-3,1-benzothiazin-2-amine (**13**). Par N-alcoylation de 600 mg (2,3 mmol) de N-méthyl-N-(4-pipéridinyl)-4H-3,1-benzothiazin-2-amine avec 674 mg de bromé **13.2** précédent fournit 795 mg (Rdt : 65 %) de composé de **formula 13 :**

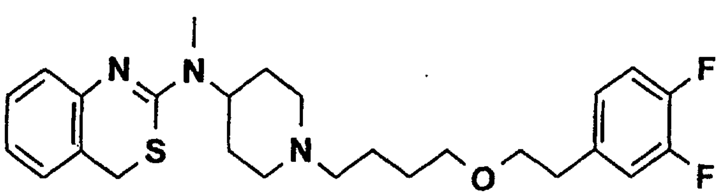

,

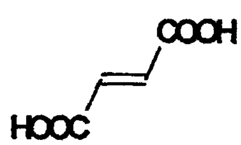

**( 13 )**

Formule brute : $C_{30}H_{37}F_2N_3O_5S$
Masse molaire : 589,71
Cristaux blanc cassé
Point de fusion : 163 °C
**RMN** (DMSO $d_6$) δ : 1,4-1,78 (m, 6H) ; 1,78-2,08 (m, 2H) ; 2,3 (t, 2H) ; 2,42-2,58 (m, 2H) ; 2,80 (t, 2H) ; 3,02 (s, 3H) ; 3-3,2 (m, 2H) ; 3,38 (t, 2H) ; 3,56 (t, 2H) ; 3,94 (s, 2H) ; 4,4 (m, 1H) ; 6,57 (s, 2H) ; 6,94 (t, 2H) ; 7,1-7,42 (m, 5H) ; 11-13 (m, 2H).

**Exemple 14 :**

**Hydrogénofumarate de la N-méthyl-N-[1-[3-[2-(3,4-difluorophényl)éthoxylpro- pyl]pipéridin-4-yl]-4H-3,1-benzothiazin-2-amine (14).**

**14.1)** 1-[2-(3-bromopropyloxy)éthyl]-3,4-difluorobenzène (**14.1**). En utilisant le procédé de l'exemple **1.1** on prépare de même avec un rendement de 32 % le composé de **formule 14.1 :**

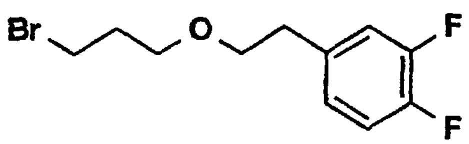

**( 14-1 )**

Formule brute : $C_{11}H_{13}BrF_2O$
Masse molaire : 279,13
Huile incolore
Point d'ébullition : 85-90 °C / 0,13 mbar
**RMN** ($CDCl_3$) δ : 2,05 (q, 2H) ; 2,7-2,9 (m, 4H) ; 3,4-3,7 (m, 4H) ; 6,8-7,2 (m, 3H).

**14.2)** Hydrogénofumarate de la N-méthyl-N-[1-[3-[2-(3,4-difluorophényl)éthoxy] propyl]pipéridin-4-yl]-4H-3,1-benzothiazin-2-amine (**14**). La condensation de 430 mg (1,53 mmol) de dérivé bromé précédent **14.1** selon le mode opératoire **1.2,** permet de préparer 512 mg (Rdt : 58 %) de dérivé de **formule 14 :**

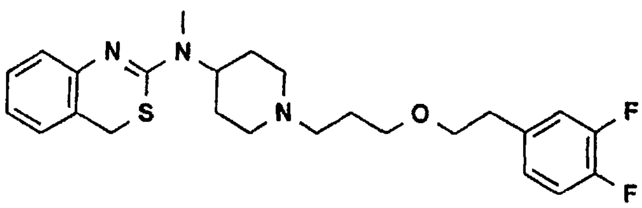
,
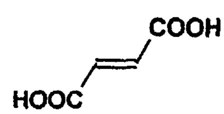

**( 14 )**

Formule brute : $C_{29}H_{35}F_2N_3O_5S$
Masse molaire : 575,68
Cristaux blancs pulvérulents
Point de fusion : 162 °C
**RMN** (DMSO $d_6$) δ : 1,55-2,1 (m, 6H) ; 2,31 (t, 2H) ; 2,4-2,57 (m, 2H) ; 2,80 (t, 2H) ; 3,02 (s, 3H) ; 3-3,18 (m, 2H) ; 3,42 (t, 2H) ; 3,57 (t, 2H) ; 3,95 (s, 2H) ; 4,4 (m, 1H) ; 6,58 (s, 2H) ; 6,94 (t, 2H) ; 7,02-7,45 (m, 5H) ; 9-11 (m, 2H).

**Exemple 15 :**

**Hydrogénofumarate de la N-méthyl-N-[1-[5-(4-méthoxyphénylméthoxy)pentyl] pipéridin-4-yl] -4H-3,1-benzothiazin-2-amine (15).**

**15.1**)1-(5-bromopentyloxyméthyl)-4-méthoxybenzène (**15.1**). Préparé selon la méthode **1.1** à partir de 2 g (15 mmol) d'alcool p-méthoxybenzylique avec un rendement de 90 % et a pour **formule 15.1 :**

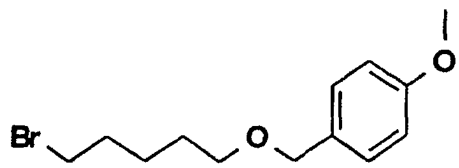

**( 15-1 )**

Formule brute : $C_{13}H_{19}BrO_2$
Masse molaire : 287,20
Huile légèrement ambrée
**RMN** ($CDCl_3$) $\delta$ : 1,4-1,75 (m, 4H) ; 1,75-1,98 (m, 2H) ; 3,32-3,5 (m, 4H) ; 3,80 (s, 3H) ; 4,44 (s, 2H) ; 6,88 (d, 2H) ; 7,26 (d, 2H).

**15.2)** Hydrogénofumarate de la N-méthyl-N-[1-[5-(4-méthoxyphénylméthoxy)pentyl] pipéridin-4-yl]-4H-3,1-benzothiazin-2-amine (**15**). Ce dérivé est préparé selon le procédé **1.2** en partant de 660 mg (2,3 mmol) de dérivé bromé précédent. On obtient 718 mg (Rdt : 53 %) de poudre de **formule 15 :**

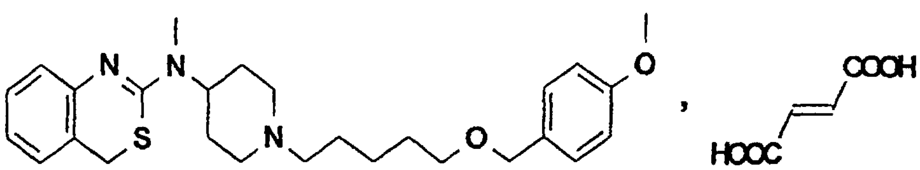

**( 15)**

Formule brute : $C_{31}H_{41}N_3O_6S$
Masse molaire : 583,75
Cristaux blancs
Point de fusion : 163 °C
**RMN** (DMSO $d_6$) $\delta$ : 1,22-1,82 (m, 8H) ; 1,84-2,1 (m, 2H) ; 2,25-2,68 (m, 4H) ; 3 (s, 3H) ; 3,08-3,24 (m, 2H) ; 3,38 (t, 2H) ; 3,74 (s, 3H) ; 3,94 (s, 2H) ; 4,3-4,55 (m, 1H) ; 4,36 (s, 2H) ; 6,56 (s, 2H) ; 6,82-7,06 (m, 4H) ; 7,08-7,32 (m, 4H) ; 12-14 (m, 2H).

**Exemple 16 :**

**Hydrogénofumarate de la N-méthyl-N-[1-[4-(4-méthoxyphénylméthoxy)butyl] pipéridin-4-yl]-4H-3,1-benzothiazin-2-amine (16).**

**16.1)** 1-(4-bromobutyloxyméthyl) -4-méthoxybenzène (**16.1**). Ce composé est préparé avec un rendement de 70 % comme décrit dans **1.1** et a pour **formule 16.1 :**

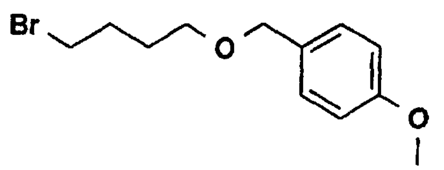

( 16-1 )

Formule brute : $C_{12}H_{17}BrO_2$
Masse molaire : 273,18
Huile incolore
**RMN** ($CDCl_3$) δ : 1,64-1,8 (m, 2H) ; 1,83-2,08 (m, 2H) ; 3,35-3,54 (m, 4H) ; 3,81 (s, 3H) ; 4,44 (s, 2H) ; 6,87 (d, 2H) ; 7,26 (d, 2H).

**16.2)** Hydrogénofumarate de la N-méthyl-N-[1-[4-(4-méthoxyphénylméthoxy)butyl] pipéridin-4-yl]-4H-3,1-benzothiazin-2-amine. La réaction de 630 mg (2,3 mmol) de dérivé bromé **16.1** ci-dessus avec 600 mg (2,3 mmol) de N-méthyl-N-(4-pipéridi- nyl)-4H-3,1-benzothiazine-2-amine donne, après salification, 849 mg (Rdt : 64 %) de composé N-alcoylé de **formula 16 :**

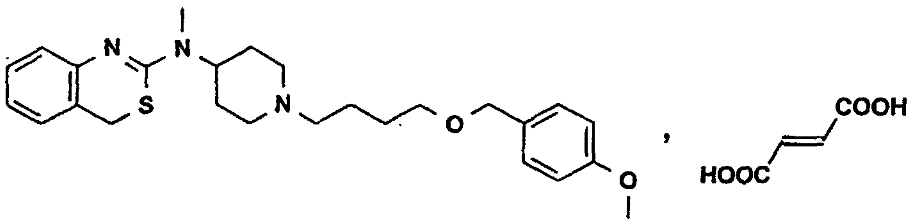

( 16 )

Formule brute : $C_{30}H_{39}N_3O_6S$
Masse molaire : 569,72
Cristaux blancs
Point de fusion : 165 °C
**RMN** (DMSO $d_6$) δ : 1,4-2,06 (m, 8H) ; 2,34 (t, 2H) ; 2,45-2,62 (m, 2H) ; 2,99 (s, 3H) ; 3,04-3,2 (m, 2H) ; 3,38 (t, 2H) ; 3,72 (s, 3H) ; 3,92 (s, 2H) ; 4,28-4,52 (m, 1H) ; 4,35 (s, 2H) ; 6,55 (s, 2H) ; 6,8-7 (m, 4H) ; 7,05-7,28 (m, 4H) ; 9-11 (m, 2H).

<u>**Exemple 17 :**</u>

**Hydrogénofumarate de la N-méthyl-N-[1-[3-[4-méthoxyphénylméthoxy)propyl] pipéridin-4-yl]-4H-3,1-benzothiazin-2-amine (17).**

**17.1)** 1-(3-bromopropyloxyméthyl)-4-méthoxybenzène (**17.1**). L'application du protocole **1.1** à 3 g (22 mmol) d'alcool 4-méthoxybenzylique donne avec un rendement de 33 % le composé de **formule 17.1 :**

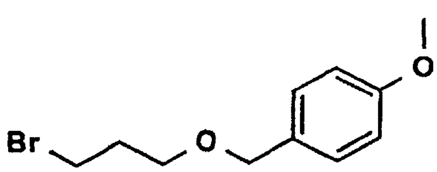

( 17-1 )

Formule brute : $C_{11}H_{15}BrO_2$
Masse molaire : 259,51
Huile légèrement ambrée

RMN ($CDCl_3$) δ : 2,11 (q, 2H) ; 3,45-3,62 (m, 4H) ; 3,79 (s, 3H) ; 4,44 (s, 2H) ; 6,86 (d, 2H) ; 7,26 (d, 2H).

a) Hydrogénofumarate de la N-méthyl-N-[1-[3-(4-méthoxyphénylméthoxy)pro- pyl]pipéridin-4-yl]-4H-3,1-benzothiazin-2-amine (17). Toujours selon le procédé de l'exemple 1.2 on prépare 910 mg (Rdt : 71 %) de composé de formule 17 à partir de 620 mg de dérivé bromé correspondant 17.1 :

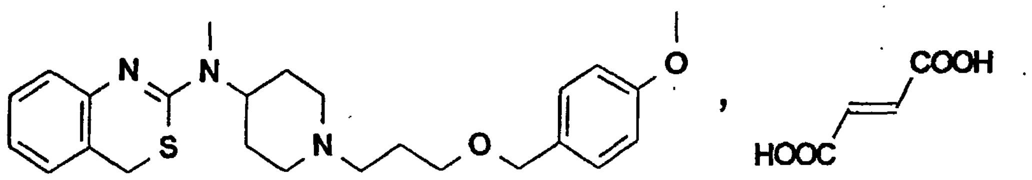

(17)

Formule brute : $C_{29}H_{37}N_3O_6S$
Masse molaire : 555,70
Cristaux blancs
Point de fusion : 166 °C
**RMN** (DMSO $d_6$) δ : 1,58-2,08 (m, 6H) ; 2,34 (t, 2H) ; 2,6 (t, 2H) ; 3,03 (s, 3H) ; 3,04-3,23 (m, 2H) ; 3,44 (t, 2H) ; 3,76 (s, 3H) ; 3,96 (s, 2H) ; 4,39 (s, 2H) ; 4,2-4,6 (m, 1H) ; 6,59 (s, 2H) ; 6,84-7,03 (m, 4H) ; 7,1-7,32 (m, 4H) ; 8,5-11 (m, 2H).

**Exemple 18 :**

**Hydrogénofumarate de la N-méthyl-N-[1-[4-[2-(4-méthoxyphényl)éthoxy]butyl] pipéridin-4-yl]-4H-3,1-benzothiazin-2-amine (18).**

**18.1)** 1-[2-(4-bromobutyloxy)éthyl]-4-méthoxybenzène (**18.1**). En opérant comme dans l'exemple **1.1** à partir de 2 g (13,1 mmol) d'alcool 4-méthoxyphénéthylique, on prépare 3,3 g (Rdt : 88 %) de dérivé bromé ayant pour **formula 18.1 :**

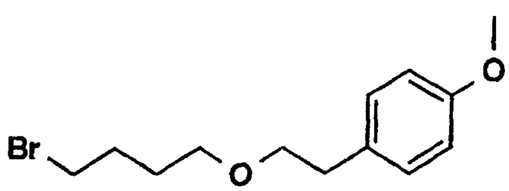

(18-1)

Formule brute : $C_{13}H_{19}BrO_2$
Masse molaire : 287,19
Huile incolore
**RMN** ($CDCl_3$) δ : 1,6-1,8 (m, 2H) ; 1,8-2 (m, 2H) ; 2,80 (t, 2H) ; 3,32-3,5 (m, 4H) ; 3,57 (t, 2H) ; 3,77 (s, 3H) ; 6,81 (d, 2H) ; 7,12 (d, 2H).
**18.2)** Hydrogénofumarate de la N-méthyl-N-[1-[4-[2-(4-méthoxyphényl)éthoxy]butyl] pipéridin-4-yl]-4H-3,1-benzothiazin-2-amine (**18**). Par application du procédé décrit dans l'exemple **1.1,** on prépare de même le composé de **formule 18,** avec un rendement de 63 % à partir de 846 mg de dérivé bromé **18.1 :**

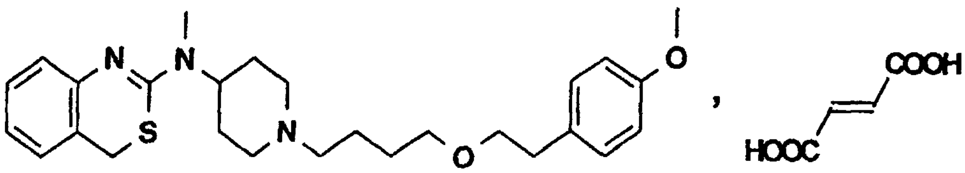

**( 18 )**

Formule brute : $C_{31}H_{41}N_3O_6S$
Masse molaire : 583,726
Cristaux blancs
Point de fusion : 156 °C
**RMN** (DMSO $d_6$) δ : 1,4-2,1 (m, 8H) ; 2,36 (t, 2H) ; 2,45-2,63 (m, 2H) ; 2,74 (t, 2H) ; 3,03 (s, 3H) ; 3,04-3,22 (m, 2H) ; 3,39 (t, 2H) ; 3,53 (t, 2H) ; 3,72 (s, 3H) ; 3,96 (s, 2H) ; 4,43 (m, 1H) ; 6,58 (s, 2H) ; 6,8-7,03 (m, 4H) ; 7,1-7,24 (m, 4H) ; 11-13 (m, 2H).

**Exemple 19 :**

**Hydrogénofumarate de la N-méthyl-N-[1-[3-[2-(4-méthoxyphényl)éthoxy]propyl] pipéridin-4-yl]-4H-3,1-benzothiazin-2-amine (19).**

**19.1)** 1-[2-(3-bromopropyloxy)éthyl]-4-méthoxybenzène (**19.1**). L'application du procédé **1.1** à l'alcool 4-méthoxyphénéthylique donne avec un rendement de 22 % le composé de **formule 19.1 :**

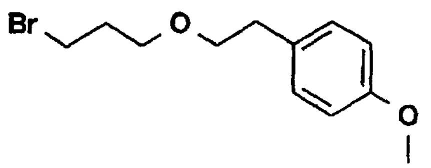

**( 19-1 )**

Formule brute : $C_{12}H_{17}BrO_2$
Masse molaire : 273,17
Huile incolore
Point d'ébullition : 103-105 °C / 0,08 mbar
**RMN** ($CDCl_3$) δ : 2,1 (q, 2H) ; 2,83 (t, 2H) ; 3,42-3,68 (m, 6H) ; 3,8 (s, 3H) ; 6,85 (d, 2H) ; 7,15 (d, 2H).

**19.2)** Hydrogénofumarate de la N-méthyl-N-[1-[3-[2-(4-méthoxyphényl)éthoxy]propyl]pipéridin-4-yl]-4H-3,1-benzothiazin-3-amine (**19**). Par réaction de 660 mg (2,4 mmol) de dérivé bromé précédent **19.1** selon le protocole **1.2** on prépare 915 mg (Rdt : 69 %) de composé de **formule 19 :**

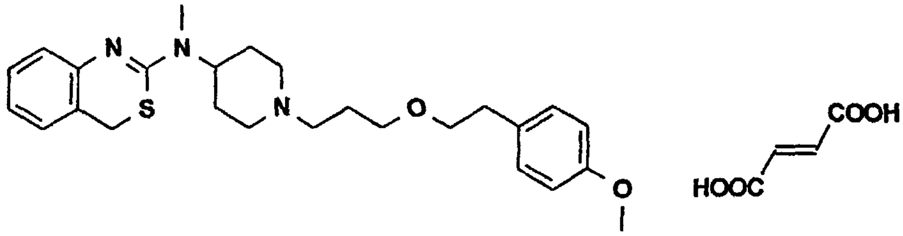

**( 19 )**

Formule brute : $C_{30}H_{39}N_3O_6S$
Masse molaire : 569,70

Cristaux blancs
Point de fusion : 155 °C
**RMN** (DMSO $d_6$) δ : 1,52-2,03 (m, 6H) ; 2,28 (t, 2H) ; 2,4-2,55 (m, 2H) ; 2,71 (t, 2H) ; 3 (s, 3H) ; 3-3,2 (m, 2H) ; 3,39 (t, 2H) ; 3,5 (t, 2H) ; 3,69 (s, 3H) ; 3,99 (s, 2H) ; 3,37 (m, 1H) ; 6,56 (s, 2H) ; 6,73-6,98 (m, 4H) ; 7,02-7,22 (m, 4H) ; 8,5-10,5 (m, 2H).

**Exemple 20 :**

**Hydrogénofumarate de la N-méthyl-N-[1-[2-[2-(4-méthoxyphényl)éthoxy]éthyl] pipéridin-4-yl]-4H-3,1-benzothiazin-2-amine (20).**

**20.1)**2-[2-(4-méthoxyphényl)éthoxy]éthanol (**20.1**). En opérant comme décrit dans l'exemple **4.1,** mais en partant de 5 g (32,8 mmol) de 2-(4-méthoxyphényl)éthanol, on prépare 2,6 g (Rdt : 40 %) de composé de **formule 20.1 :**

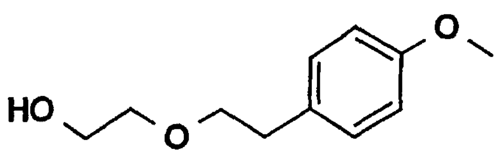

( 20-1 )

Formule brute : $C_{11}H_{16}O_3$
Masse molaire : 196,24
Huile incolore
**RMN** ($CDCl_3$) δ : 1,89 (s, 1H) ; 2,87 (t, 2H) ; 3,58 (t, 2H) ; 3,63-3,75 (m, 4H) ; 3,81 (s, 3H) ; 6,86 (d, 2H) ; 7,36 (d, 2H).

**20.2)** Mésylate de 2- [2- (4-méthoxyphényl) éthoxy]éthyle (**20.2**). La réaction de 0,52 ml (6,7 mmol) de chlorure de mésyle, sur 1,2 g d'alcool **20.1** précédent selon **4.2,** donne 1,56 g (Rdt : 93 %) de composé de **formule 20.2 :**

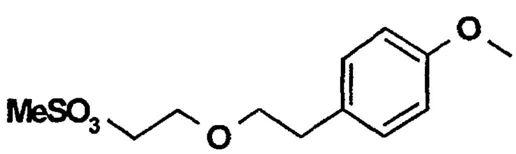

( 20-2 )

Formule brute : $C_{12}H_{18}O_5S$
Masse molaire : 274,32
Liquide jaune clair
**RMN** ($CDCl_3$) δ : 2,86 (t, 2H) ; 2,96 (s, 3H) ; 3,65-3,75 (m, 4H) ; 3,80 (s, 3H) ; 4,32-4,42 (m, 2H) ; 6,85 (d, 2H) ; 7,15 (d, 2H).

**20.3)** Hydrogénofumarate de la N-méthyl-N- [1-[2-[2-(4-méthoxy-phényl)éthoxy]éthyl]pipéridin-4-yl]-4H-3,1-benzothiazin-2-amine (20). L'application du procédé 1.2 à 800 mg (2,9 mmol) de mésylate précédent **20.2,** donne 615 mg (Rdt : 41 %) de composé de **formule 20 :**

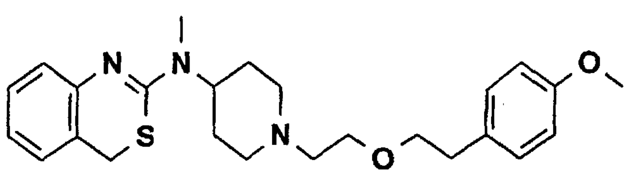

,

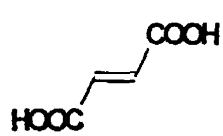

( 20 )

Formule brute : $C_{29}H_{37}N_3O_6S$
Masse molaire : 555,67
Cristaux blancs
Point de fusion : 152-3 °C
**RMN** (DMSO $d_6$) δ : 1,5-1,63 (m, 2 H) ; 1,75-1,92 (m, 2H) ; 2,52 (t, 2H) ; 2,63 (t, 2H) ; 2,74 (t, 2H) ; 3,02 (s, 3H) ; 3-31 (m, 2H) ; 3,5-3,6 (m, 4H) ; 3,70 (s, 3H) ; 3,94 (s, 2H) ; 4,33 (m, 1H) ; 6,6 (s, 2H) ; 6,84 (d, 2H) ; 6,93 (q, 2H) ; 7,1-7,2 (m, 4H) ; 10-12 (m, 2H).

__Exemple 21 :__

**Hydrogénofumarate de la N-méthyl-N-[1-(2-phénylméthoxyéthyl)pipéridin-4-yl] -4H-3,1-benzothiazin-2-amine (21).**

**21.1)** Mésylate de 2-phénylméthoxyéthyle (**21.1**). En partant de 2 g (13,1 mmol) de 2-benzyloxyéthanol en adaptant le procédé décrit dans l'exemple **4.2,** on prépare 3,02 g (Rdt : 94 %) d'une huile de **formule 21.1 :**

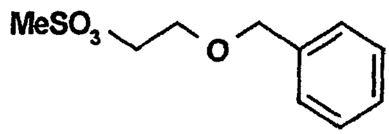

**( 21-1 )**

Formule brute : $C_{10}H_{14}O_4S$
Masse molaire : 230,27
Huile jaune clair
**RMN** ($CDCl_3$) δ : 3,02 (s, 3H) ; 3,65-3,77 (m, 2H) ; 4,34-4,43 (m, 2H) ; 4,56 (s, 2H) ; 7,32 (s, 5H).

**21.2)** Hydrogénofumarate de la N-méthyl-N-[1-(2-phénylméthoxyéthyl)pipéridin-4-yl]-4H-3,1-benzothiazin-2-amine (**21**). L'action de 580 mg (2,5 mmol) de mésylate **21.1** précédent, sur 600 mg (2,3 mmol) de N-méthyl-N-[4-pipéridinyl)-4H-3,1-benzothiazin-2-amine selon le procédé **1.2,** donne 870 mg (Rdt : 74 %) de poudre de **formule 21 :**

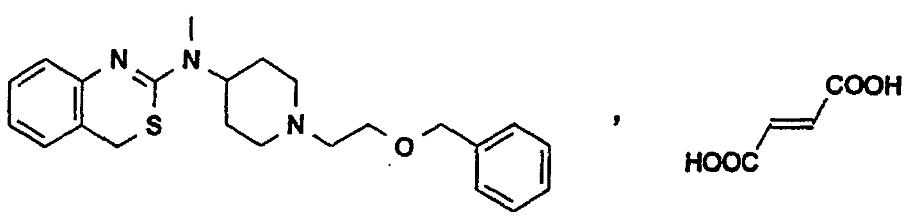

**( 21 )**

Formule brute : $C_{27}H_{33}N_3O_5S$
Masse molaire : 511,62
Cristaux blancs
Point de fusion : 166-7 °C
**RMN** (DMSO $d_6$) δ : 1,52-2,02 (m, 4H) ; 2,32 (t, 2H) ; 2,71 (t, 2H) ; 3 (s, 3H) ; 3-3,17 (m, 2H) ; 3,59 (t, 2H) ; 3,93 (s, 2H) ; 4,35 (m, 1H) ; 4,83 (s, 2H) ; 6,58 (s, 2H) ; 6,92 (t, 2H) ; 7,09-7,2 (m, 2H) ; 7,2-7,4 (m, 5H) ; 11-13 (m, 2H).

__Exemple 22 :__

**Hydrogénofumarate de la N-méthyl-N-[1-[3-(2-phényléthoxy)propyl]pipéridin-4-yl]-4H-3,1-benzothiazin-2-amine (22).**

**22.1)** 1-(2-phényléthoxy) -3-chloropropane (**22.1**). En opérant à partir de 20 g (0,164 mmol) d'alcool phénéthylique

selon le procédé décrit dans l'exemple **6.1**, on prépare 24,32 g (Rdt : 75 %) de composé de **formule 22.1 :**

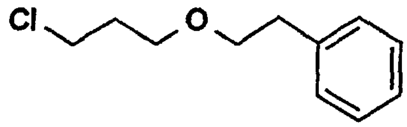

**( 22-1 )**

Formule brute : $C_{11}H_{15}ClO$
Masse moléculaire : 198,68
Huile incolore
Point d'ébullition : 85-90 °C / 0,15 mbar
**RMN** ($CDCl_3$) δ: 2,07 (q, 2H) ; 2,88 (t, 2H); 3,5-3,68 (m, 4H); 7,17-7,3 (m, 5H).

**22.2)** Hydrogénofumarate de la N-méthyl-N- [1- [3- (2-phényléthoxy)propyl]pipéridin-4-yl]-4H-3,1-benzothiazin-2-amine (**22**). Par condensation de 523 mg (2,63 mmol) de dérivé chloré précédent (**22.1**), sur 665 mg (2,51 mmol) de N-méthyl-N-(4-pipéridinyl)-4H-3,1-benzothiazin-2-amine, selon le protocole décrit dans l'exemple **1.2** on prépare 698 mg (Rdt : 51 %) de composé de formule :

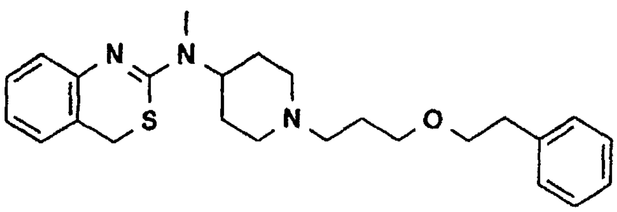

,

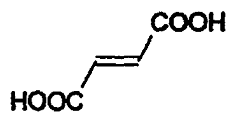

**( 22 )**

Formule brute : $C_{29}H_{37}N_3O_5S$
Masse molaire : 539,67
Cristaux blancs pulvérulents
Point de fusion : 166 °C
**RMN** (DMSO $d_6$) δ : 1,6-1,73 (m, 4H) ; 1,78-1,92 (m, 2H) ; 2,17 (t, 2H) ; 2,45 (t, 2H) ; 2,8 (t, 2H) ; 3,02 (s, 3H) ; 3-3,1 (m, 2H) ; 3,42 (t, 2H) ; 3,57 (t, 2H) ; 3,94 (s, 2H) ; 4,35 (m, 1H) ; 6,59 (s, 2H) ; 6,88-6,97 (m, 2H) ; 7,1-7,5 (m, 7H) ; 12-14 (m, 2H).

**Exemple 23 :**

**Hémifumarate de la N-méthyl-N-[1-[2-hydroxy-3-(4-fluorophénylméthoxy)propyl]pipéridin-4-yl] -4H-3,1-benzothiazin-2-amine (23).**

**23.1)** 1-(4-fluorophénylméthoxy)-2,3-époxypropane (**21.1**). Un mélange de 55 ml de lessive de soude à 50 % (en poids), 1,38 g (4 mmol) d'hydrogénosulfate de tétrabutyl ammonium et 78 ml d'épichlorhydrine est agité fortement à 25 °C, puis traité en 10-15 minutes (goutte à goutte) avec 12,7 g (0,1 mol) d'alcool 4-fluoroben-zylique en maintenant la température inférieure à 28°C. L'agitation est poursuivie 3h30 de plus, puis le mélange est extrait à l'éther, lavé au bicarbonate de sodium, à l'eau, à l'eau salée et séché sur sulfate de sodium anhydre. Après élimination du sel minéral, le filtrat est évaporé à siccité pour donner une huile pâle qui est rectifiée sous vide et de **formule 23.1** (Rdt : 75 %) :

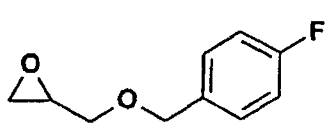

**( 23-1 )**

Formule brute : $C_{10}H_{11}FO_2$
Masse molaire : 182,19
Huile incolore
Point d'ébullition : 90-92 °C / 0,15 mbar
**RMN** ($CDCl_3$) δ : 2,62 (q, 1H) ; 2,81 (t, 1H) ; 3,15-3,25 (m, 1H) ; 3,32-3,48 (m, 1H) ; 3,78 (dd, 1H) ; 4,55 (q, 2H) ; 7,03 (t, 2H) ; 7,22-7,34 (m, 2H).

**23.2)** Hémifumarate de la N-méthyl-N-[1-[2-hydroxy-3-(4-fluorophényl)méthoxy)propyl]pipéridin-4-yl]-4H-3,1-benzothiazin-2-amine (**23**). Une solution de 580 mg (3,2 mmol) d'époxyde précédent **23.1** dans 8 ml d'éthanol est traitée avec 800 mg (3,06 mmol) de N-méthyl-N-(4-pipéridinyl)-4H-3,1-benzothiazin-2-amine et agitée une nuit à 25 °C. Le mélange réactionnel est évaporé à siccité puis le résidu est purifié par flash chromatographie en éluant avec un mélange $CH_2Cl_2$/ $CH_3OH$//$NH_4OH$ - 97,5/2,25/0,25, pour donner une huile jaune clair (m = 1,20 g ; Rdt : 85 %).
La base précédente est salifiée de la manière habituelle. L'hémifumarate de **formule 23** précipite (m = 1,05 g ; Rdt global : 68 %).

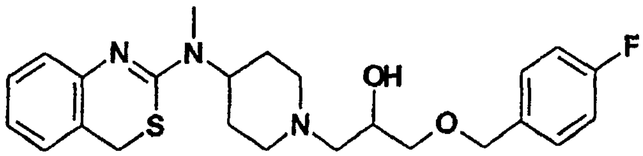

, 1/2

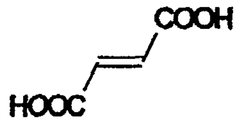

**(23)**

Formule brute : $C_{26}H_{32}FN_3O_4S$
Masse molaire : 501,60
Cristaux blancs pulvérulents
Point de fusion : 155-156 °C
**RMN** (DMSO $d_6$) δ : 1,5-1,63 (m, 2H) ; 1,75-1,85 (m, 2H) ; 2,17-2,3 (m, 2H) ; 2,3-2,52 (m, 2H) ; 3,02 (s, 3H) ; 3-3,09 (m, 2H) ; 2,8-3,5 (m, 1H) ; 3,3-3,47 (m, 2H) ; 3,75-3,85 (m, 1H) ; 3,94 (s, 2H) ; 4,32 (m, 1H) ; 4,48 (s, 2H) ; 6,58 (s, 1H) ; 6,85-6,98 (m, 2H) ; 7,1-7,23 (m, 4H) ; 7,35-7,42 (m, 2H) ; 11-13 (m, 1H).

**Exemple 24 :**

**Hydrogénofumarate de la N-méthyl-N-[1-[2-hydroxy-3-[2-(4-méthoxyphényl) éthoxy]propyl]pipéridin-4-yl]-4H-3,1-benzothiazin-2-amine (24).**

**24.1)** 1-[2- (4-méthoxyphényl)éthoxy]-2,3-époxypropane (**24.1**). Par condensation de 4,8 g (31,5 mmoles) d'alcool 4-méthoxyphénéthylique dans un mélange de 25 ml d'épichlorhydrine et 18 ml de lessive de soude à 50 % et 430 mg de $Bu_4NHSO_4$ selon le procédé **23.1** on prépare 4,42 g (Rdt : 6 7 %) d'éther glycidique de **formule 24.1 :**

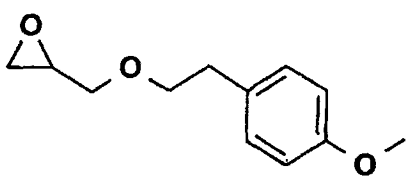

**(24-1)**

Formule brute : $C_{12}H_{16}O_3$
Masse moléculaire : 208,25
Huile incolore
Point d'ébullition : 130-135 °C / 0,25 mbar
**RMN** ($CDCl_3$) δ : 2,6 (s, 1H) ; 2,78 (s, 1H) ; 2,79-2,91 (m, 2H) ; 3 (s, 1H) ; 3,34-3,5 (m, 1H) ; 3,5-3,89 (m, 3H) ; 3,9 (s, 3H) ; 6,83 (d, 2H) ; 7,14 (d, 2H).

**24.2)** Hydrogénofumarate de la N-méthyl-N-[1-[2-hydroxy-3-[2-(4-méthoxyphényl)éthoxy]propyl]pipéridin-4-yl]-4H-3,1-benzothiazin-2-amine (**24**). En utilisant le pro- cédé de l'exemple **23.2** mais en partant de 620 mg (2,9 mmol) d'éthyl glycidique (**24.1**) précédent, on prépare 692 mg (Rdt : 41 %) de composé de **formule 24 :**

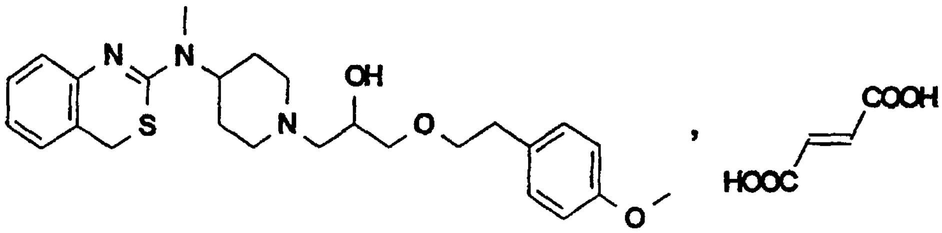

**(24)**

Formule brute : $C_{30}H_{39}N_3O_7S$
Masse molaire : 585,70
Cristaux blanc cassé
Point de fusion : 132-3 °C
**RMN** (DMSO $d_6$) δ : 1,5-1,67 (m, 2H) ; 1,8-1,98 (m, 2H) ; 2,25-2,48 (m, 4H) ; 2,74 (t, 2H) ; 3,02 (s, 3H) ; 3,08 (t, 2H) ; 2,9-3,2 (m, 1H) ; 3,3-3,39 (m, 2H) ; 3,57 (t, 2H) ; 3,71 (s, 3H) ; 3,73-3,78 (m, 1H) ; 3,94 (s, 2H) ; 4,36 (m, 1H) ; 6,59 (s, 2H) ; 6,84 (d, 2H) ; 6,88-6,97 (m, 2H) ; 7,06-7,21 (m, 4H) ; 10-12 (m, 2H).

__Exemple 25 :__

**Hydrogénofumarate de la 6,N-diméthyl-N-[1-[3-[2-(4-fluorophenyl)éthoxy]propyl]pipéridin-4-yl]-4H-3,1-benzothiazin-2-amine (25) .**

**25.1)**Alcool 2-amino-5-méthylbenzylique (**25.1**) : Une suspension de 3,80 g (0,1 mol) de $LiAlH_4$ dans 200 ml de THF sec refroidie sur bain de glace, est traitée goutte à goutte avec une solution de 17,95 g (0,1 mol) de 2-amino-5-méthyl benzoate d'éthyle dissous dans 150 ml de THF. On agite 1h de plus à cette température, puis laisse revenir à 25 °C et maintient l'agitation 4h de plus à 25 °C. Le mélange est refroidi à 0 °C et traité goutte à goute avec 30 ml d'acétate d'éthyle, puis on ajoute 10 ml d'eau goutte à goutte, sèche sur sulfate de sodium anhydre et filtre l'insoluble. Le mélange est évaporé à siccité pour donner une huile ambrée, qui est triturée dans l'éther isopropylique pour donner 9,42 g (Rdt : 68 % ) de poudre de formule **25.1 :**

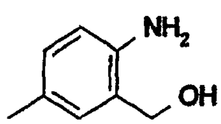

**( 25-1 )**

Formule brute : $C_8H_{11}NO$
Masse moléculaire : 137,18
Poudre beige
Point de fusion : 124 °C
**RMN** (DMSO $d_6$) δ : 2,13 (s, 3H) ; 4,33 (d, 2H) ; 4,68(s, 2H) ; 4,95 (t, 1H) ; 6,51 (d, 1H) ; 6,7-6,8 (m, 1H) ; 6,86 (s, 1H).

**25.2)** 1-[3-[2-(4-fluorophényl)éthoxy]propyl]-4-pipéridone (**25.2**). Une solution de 17,5 g (81 mmol) de 1-fluoro-4-[2-(3-chloropropyloxy)éthyl]benzène (préparé selon l'exemple **22.1**) dans 175 ml de DMF, est traitée sous agitation à 25 °C avec 10,4 ml de 1,4-dioxa-8-azaspiro[4,5]décane (11,6 g ou 81 mmol) et 12,4 g (85 mmol) d'un mélange intimement broyé de $K_2CO_3$/KI, 98/02, puis le mélange est chauffé pendant 5h à 80 °C. L'insoluble est éliminé par filtration et la solution obtenue est évaporée à siccité sous vide. Le résidu est formé par l'aminocétone protégée de formule **25.2.1** qui n'est pas isolée.

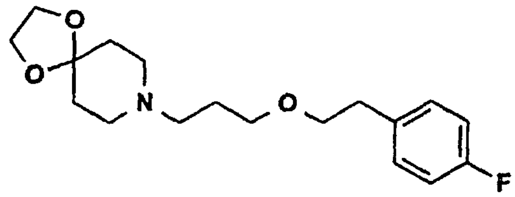

( 25-2-1 )

Ce composé est traité avec 160 ml d'acide chlorhydrique 6N et chauffé 2 h à 100 °C sous agitation. Après retour à 25 °C, on extrait les insalifiables au $CH_2Cl_2$ et la phase aqueuse est séparée, refroidie à 0 °C et alcalinisée avec de la soude 10N jusqu'à pH 12-13. L'amino cétone est extraite au $CH_2Cl_2$, puis on lave à l'eau, à l'eau salée, sèche sur sulfate de sodium anhydre, filtre et évapore à siccité : on obtient 17,3 g de résidu brut de **formule 25.2 :**

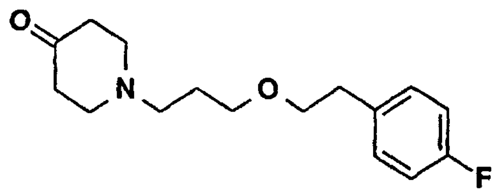

( 25-2 )

Formule brute : $C_{16}H_{22}FNO_2$
Masse moléculaire : 279,35
Huile visqueuse légèrement orangée
**RMN** ($CDCl_3$) $\delta$ : 1,5-1,85 (m, 2H) ; 2,36-2,58 (m, 6H) ; 2,72 (t, 4H) ; 2,85 (t, 2H) ; 3,5 (t, 2H) ; 3,61 (t, 2H) ; 6,91-7 (m, 2H) ; 7,1-7,2 (m, 2H).

**25.3)** Dichlorhydrate de la 4-méthylamino-1-[3-[2-(4-fluorophényl)éthoxy]propyl) pipéridine (**25.3**). Une solution de 14,7 g (52,6 mmol) de cétoamine précédente (**25.2**) dans 150 ml de $CH_2Cl_2$, est traitée avec 3,5 g (52,6 mmol) de chlorhydrate de méthylamine, puis on ajoute du méthanol jusqu'à dissolution totale. Après 3h d'agitation à 25 °C, le mélange est refroidi sur bain de glace et on ajoute par fraction 14,5 g (68,5 mmol) de triacétate de borohydrure de sodium et enfin 3 ml d'acide acétique goutte à goutte. L'agitation est poursuivie une nuit à 25 °C. Le mélange réactionnel est versé sur de la glace et alcalinisé jusqu'à pH 12-13. La base libérée est extraite de la manière habituelle pour donner une huile verdâtre.
Après dissolution dans 70 ml d'éthanol absolu, on chlorhydrate à 0 °C par addition d'une solution éthanolique chlorhydrique 2,5 N. Le sel cristallise après amorçage et est récupéré par filtration pour donner 13,6 g (Rdt : 70 %) de cristaux de **formule 25.3 :**

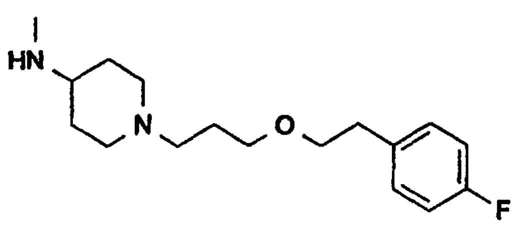

, 2 HCl ( 25-3 )

Formule brute : $C_{17}H_{29}Cl_2FN_2O$
Masse molaire : 367,34
Cristaux blanc cassé
**RMN** (DMSO $d_6$) $\delta$ : 1,9-2,05 (m, 4H) ; 2,12-2,3 (m, 2H) ; 2,50 (s, 3H) ; 2,8 (t, 2H) ; 2,84-3 (m, 4H) ; 3,17 (m, 1H) ; 3,45 (t, 2H) ; 3,5-3,6 (m, 4H) ; 7,11 (t, 2H) ; 7,28 (dd, 2H) ; 9,42 (m, 2H) ; 10,7 (m, 1H).

**25.4)**Chlorhydrate de la 1-[1-[3-[2-(4-fluorophényl) éthoxy]propyl]pipéridin-4-yl]-1-méthyl-3-(2-hydroxyméthyl-4-méthylphényl)thiourée (**25.4**). A une suspension de 700 mg (5 mmol) de $K_2CO_3$ dans 10 ml de $CH_2Cl_2$ refroidie sur bain de glace et placée sous courant d'azote, on ajoute 0,39 ml (580 mg, 5 mmoles) de thiophosgène, puis

une solution de 1,47 g (5 mmol) de diamine précédente (**25.3** sous forme de base libre) goutte à goutte en 20 minutes. Après une heure d'agitation supplémentaire, l'insoluble est éliminé par filtration et le filtrat renfermant le composé intermédiaire **25.4.1**) :

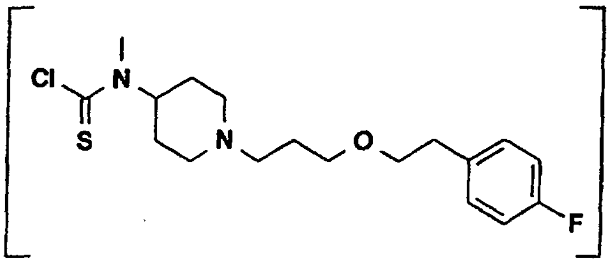

**( 25-4-1 )**

est ajouté goutte à gouttte à une solution de 690 mg (5 mmol) d'alcool 2-amino-5-méthylbenzylique dans 30 ml de $CH_2Cl_2$ et l'agitation est poursuivie pendant 1h. Le mélange réactionnel est évaporé à siccité pour donner un résidu qui, trituré dans l'éther, donne 1,86 g (Rdt : 73 %) de composé de **formule 25.4 :**

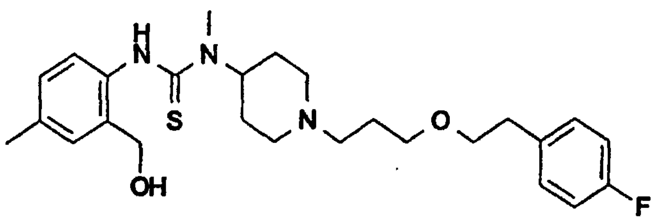

, HCl **( 25-4 )**

Formule brute : $C_{26}H_{37}ClFN_3O_2S$
Masse moléculaire : 510,09
Cristaux crème
Point de fusion : 112-115 °C
**RMN** ($CDCl_3$) $\delta$ : 1,9-2,13 (m, 4H) ; 2,33 (s, 3H) ; 2,5-2,68 (m, 2H) ; 2,72-2,85 (m, 4H) ; 2,89-2,98 (m, 2H) ; 3,2 (s, 3H) ; 3,40-3,63 (m, 5H) ; 3,88 (t, 2H) ; 4,58 (s, 2H) ; 5,73 (m, 1H) ; 6,9-7,03 (m, 3H) ; 7,08-7,24 (m, 4H) ; 7,53 (d, 1H) ; 8,31 (s, 1H) ; 12 (m, 1H).

**25.5)** Hydrogénofumarate de la 6,N-diméthyl-N-[1-[3-[2-(4-fluorophényl)éthoxy] propyl]pipéridin-4-yl]-4H-3,1-benzothiazin-2-amine (**25**). Un mélange de 1,75 g (3,43 mmol) d'hydroxythiourée précédente (**25.4**) dans 4 ml d'acide chlorhydrique concentré est chauffé à 50 °C pendant 20 minutes. Le mélange réactionnel est ensuite versé sur de la glace pilée et alcalinisé avec de la soude jusqu'à pH 10-12, puis extrait à l'acétate d'éthyle, lavé à l'eau, à l'eau salée, séché sur sulfate de sodium anhydre, filtré et évaporé à siccité pour donner 1,39 g (Rdt : 82 %) d'une huile qui cristallise, de **formule 25.5 :**

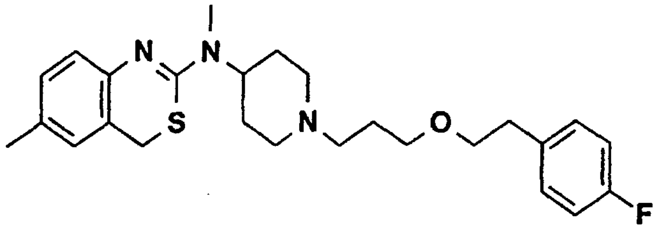

**( 25-5 )**

Formule brute : $C_{26}H_{34}FN_3OS$
Masse moléculaire : 455,62
Cristaux beiges

Point de fusion : 70 °C
**RMN** ($CDCl_3$) δ : 1,65-1,93 (m, 6H) ; 2-2,1 (m, 2H) ; 2,3 (s, 3H) ; 2,39 (t, 2H) ; 2,84 (t, 2H) ; 2,92-2,98 (m, 2H) ; 3,01 (s, 3H) ; 3,47 (t, 2H) ; 3,6 (t, 2H) ; 3,81 (s, 2H) ; 4,37 (m, 1H) ; 6,87 (s, 1H) ; 6,92-7,05 (m, 4H) ; 7,12-7,2 (m, 2H).

Le fumarate est réalisé de la manière habituelle à partir de 1,16 g de base précédente dans l'éthanol pour donner 1,21 g de composé de **formule 25 :**

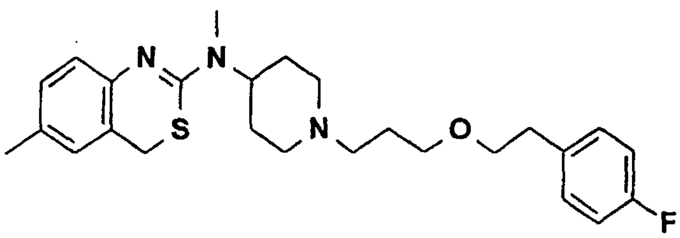
,
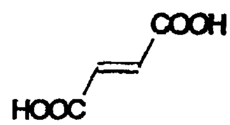

**(25)**

Formule brute : $C_{30}H_{38}FN_3O_5S$
Masse moléculaire : 571,69
Cristaux blancs
Point de fusion : 169-172 °C
**RMN** (DMSO $d_6$) δ : 1,58-1,75 (m, 4H) ; 1,8-1,93 (m, 2H) ; 2,18-2,29 (m, 5H) ; 2,43-2,51 (m, 2H) ; 2,79 (t, 2H) ; 2,99 (s, 3H) ; 3-3,09 (m, 2H) ; 3,41 (t, 2H) ; 3,55 (t, 2H) ; 3,9 (s, 2H) ; 4,34 (m, 1H) ; 6,58 (s, 2H) ; 6,8 (d, 1H) ; 6,93-7 (m, 2H) ; 7,1 (t, 2H) ; 7,25-7,3 (m, 2H) ; 12-14 (m, 2H).

<u>**Exemple 26 :**</u>

**Hydrogénofumarate de la 6-chloro-N-méthyl-N-[1-[3-[2-(4-fluorophényl)éthoxy]propyl]pipéridin-4-yl]-4H-3,1-benzothiazin-2-amine (26).**

**26.1)** 1-[1-[3-[2-(4-fluorophényl)éthoxy]propyl]pipéridin-4-yl]-1-méthyl-3-[4-chloro-2-hydroxyméthyl]phényl)thiourée (**26.1**). En opérant comme décrit dans l'exemple **25.4,** mais en partant de 880 mg (3 mmol) de diamine **25.3,** on prépare la solution de chlorure de thiocarbamoyle **25.4.1**, qui est ajoutée à une solution de 470 mg (3,7 mmol) d'alcool 2-amino-5-chlorobenzylique. L'hydroxy thiourée est dans ce cas purifiée par flash chromatographie, pour donner une huile légèrement colorée (m = 1,1 g ; Rdt : 74 %) de **formula 26.1 :**

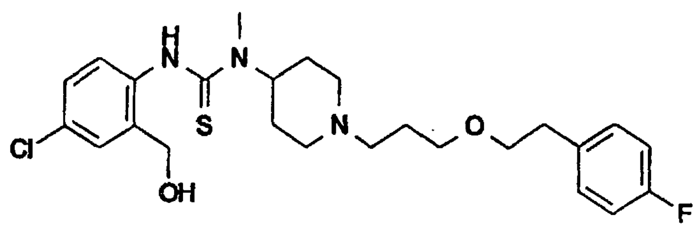

**(26-1)**

Formule brute : $C_{25}H_{33}ClFN_3O_2S$
Masse moléculaire : 494 ,05
Huile ambrée
**RMN** ($CDCl_3$) δ : 1,5-1,9 (m, 6H) ; 2,02-2,15 (m, 2H) ; 2,39 (t, 2H) ; 2,84 (t, 2H) ; 2,92-3 (m, 2H) ; 3,11 (s, 3H) ; 3,44 (t, 2H) ; 3,6 (t, 2H) ; 4,59 (s, 2H) ; 5,18 (m, 1H) ; 6,95-7 (m, 2H) ; 7,13-7,22 (m, 2H) ; 7,23-7,33 (m, 2H) ; 7,72 (d, 1H) ; 7,98 (s, 1H).

**26.2)** Hydrogénofumarate de la 6-chloro-N-méthyl-N-[1-[3-[2-(4-fluorophényl)étho-xy]propyl]pipéridin-4-yl]-4H-3,1-benzothiazin-2-amine (**26**). La cyclisation de 750 mg (1,52 mmol) de base précédente **26.1,** dans 4 ml d'acide

chlorhydrique concentré selon le protocole de l'exempl **25.5,** donne 690 mg (Rdt : 96 %) de poudre de **formule 26.2 :**

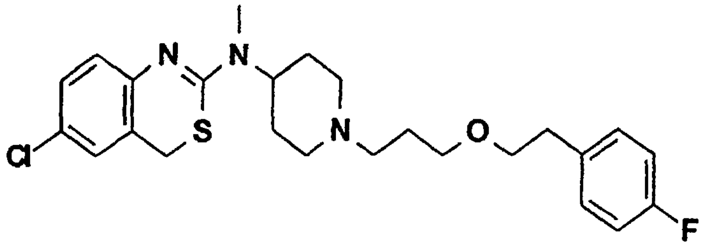

**(26-2)**

Formule brute : $C_{25}H_{31}ClFN_3OS$
Masse moléculaire : 476,04
Cristaux blancs
Point de fusion : 72 °C
**RMN** ($CDCl_3$) δ : 1,63-1,92 (m, 6H) ; 2-2,09 (m, 2H) ; 2,32-2,44 (m, 2H) ; 2,8-2,87 (m, 2H) ; 2,93-3,02 (m, 2H) ; 3,09 (s, 3H) ; 3,47 (t, 2H) ; 3,55-3,63 (m, 2H) ; 3,81 (s, 2H) ; 4,38 (m, 1H) ; 6,92-7 (m, 3H) ; 7,05 (d, 1H) ; 7,12-7,2 (m, 3H).
La base précédente salifiée de la manière habituelle donne 695 mg de cristaux (Rdt global : 78 %) de **formule 26 :**

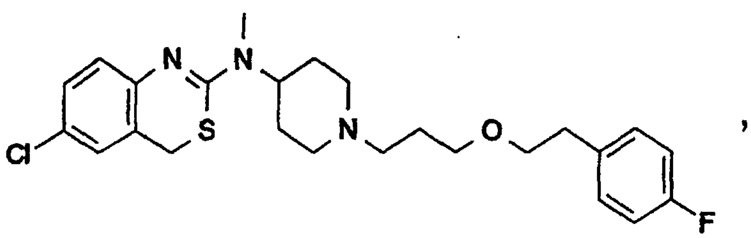

,

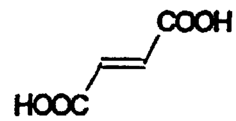

**(26)**

Formule brute : $C_{29}H_{35}ClFN_3O_5S$
Masse moléculaire : 592,11
Cristaux blancs
Point de fusion : 167-170 °C
**RMN** (DMSO $d_6$) δ : 1,6-1,72 (m, 4H) ; 1,6-1,72 (m, 2H) ; 2,15 (t, 2H) ; 2,42 (t, 2H) ; 2,79 (t, 2H) ; 2,95-3,05 (m, 5H) ; 3,41 (t, 2H) ; 3,55 (t, 2H) ; 3,96 (s, 2H) ; 4,33 (m, 1H) ; 6,58 (s, 2H) ; 6,90 (d, 1H) ; 7,10 (t, 2H) ; 7,15-7,31 (m, 4H) ; 13 (m, 2H).

**Exemple 27 :**

**Hydrogénofumarate de la N-méthyl-N-[1-[3-[2-(4-fluorophényl) éthoxy]propyl] pipéridin-4-yl]-6,7,8-triméthoxy-4H-3,1-benzothiazin-2-amine (27).**

**27.1)**alcool 2-amino-3,4,5-triméthoxybenzylique (**27.1**). Par réduction de 12,06 g (50 mmoles) de 3,4,5-triméthoxy anthranilate de méthyle selon le protocole de l'exemple **25.1,** on prépare avec un rendement de 82 % le composé de **formule 27.1 :**

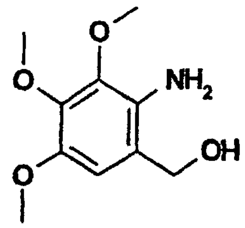

**( 27-1 )**

Formule brute : $C_{10}H_{15}NO_4$
Masse moléculaire : 213,23
Cristaux beiges
Point de fusion : 56-58 °C

**27.2)** 1-[1-[3-[2-(4-fluorophényl)éthoxy]propyl]pipéridin-4-yl]-1-méthyl-3-(6-hydro-xyméthyl-2,3,4-triméthoxyphényl)thiourée (**27.2**). En partant de 880 mg (3 mmol) de diamine **25.3**, on prépare comme décrit dans l'exemple **25.4**, le chlorure thiocarbamoyle intermédiaire qui est condensé sur 640 mg (3 mmol) d'alcool 2-amino-3,4,5-triméthoxybenzylique **27.1** et donne après purification par flash chromatographie 1,22 g (Rdt : 73 %) d'une huile de **formule 27.2 :**

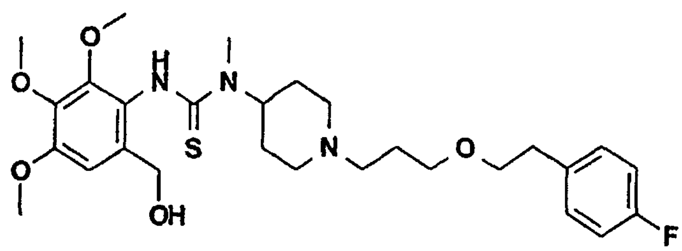

**( 27-2 )**

Formule brute : $C_{28}H_{40}FN_3O_5S$
Masse moléculaire : 549,69
Huile visqueuse ambrée
**RMN** ($CDCl_3$) $\delta$ : 1,65-1,85 (m, 6H) ; 2,06-2,16 (m, 2H) ; 2,35-2,45 (m, 2H) ; 2,84 (t, 2H) ; 2,95-3,03 (m, 2H) ; 3,19 (s, 3H) ; 3,46 (t, 2H) ; 3,6 (t, 2H) ; 3,84-3,92 (m, 11H) ; 4,51 (m, 1H) ; 5,19 (m, 1H) ; 6,63 (s, 1H) ; 6,84 (s, 1H) ; 6,92-7 (m, 2H) ; 7,14-7,21 (m, 2H).

**27.3**) Hydrogénofumarate de la N-méthyl-N-[1-[3-[2-(4-fluorophényl)éthoxy]propyl] pipéridin-4-yl]-6,7,8-triméthoxy-4H-3,1-benzothiazin-2-amine (**27**). En réalisant la cyclisation de 950 mg (2,73 mmol) d'hydroxy thiourée précédente **27.2**, selon le procédé décrit dans l'exemple **25.5,** on prépare 698 mg (Rdt : 62 %) de poudre blanche de **formule 27 :**

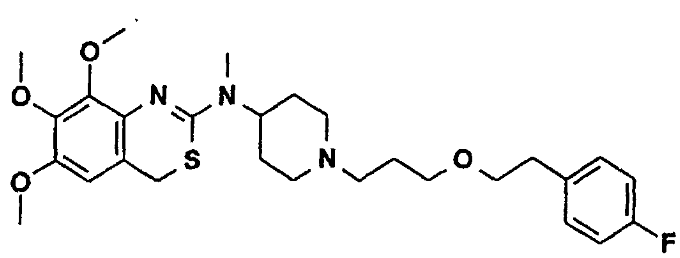

**,**

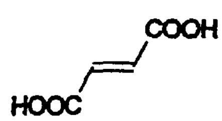

**( 27 )**

Formule brute : $C_{32}H_{42}FN_3O_8S$
Masse moléculaire : 677,74
Cristaux beiges pulvérulents
Point de fusion : 68 °C

**RMN** (DMSO $d_6$) δ : 1,62-1,73 (m, 4H) ; 1,8-1,92 (m, 2H) ; 2,19 (t, 2H) ; 2,46 (t, 2H) ; 2,79 (t, 2H) ; 3,01 (s, 3H) ; 3-3,08 (m, 2H) ; 3,41 (t, 2H) ; 3,55 (t, 2H) ; 3,69 (s, 3H) ; 3,74 (s, 3H) ; 3,83 (s, 3H) ; 3,87 (s, 2H) ; 4,39 (m, 1H) ; 6,58 (s, 2H) ; 6,62 (s, 1H) ; 7,1 (t, 2H) ; 7,27 (t, 2H) ; 12-14 (m, 2H).

__Exemple 28 :__

**Dihydrogénofumarate de la N-[1-[3-[2-(3,4-difluorophényl)éthoxy]propyl]pipéridin-4-yl]-4H-3,1-benzothiazin-2-amine (28).**

Par condensation de 910 mg (3,23 mmol) de 1-[2-(3-bromopropoxy)éthyl]-3,4-difluorobenzène (préparé dans l'exemple **14.1**) sur 800 mg (3,23 mmol) de N-(4-pipéridinyl)-4H-3,1-benzothiazin-2-amine (préparé comme décrit dans l'application WO 97-05134) en présence de 385 mg (3,56 mmol) de $K_2CO_3$/KI 98/02 dans 8 ml de DMF selon le protocole de l'exemple **1.2,** on prépare après addition de 2 équivalents d'acide maléique, 985 mg (Rdt : 45 %) de poudre de **formule 28 :**

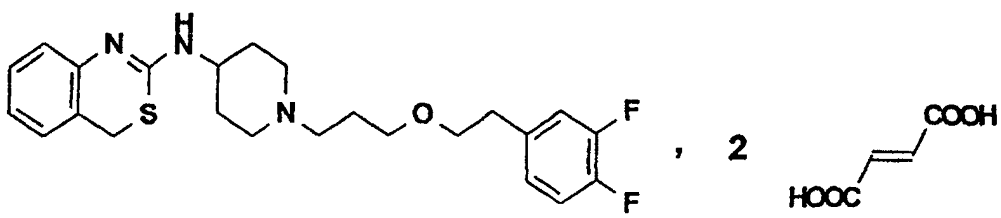

, 2

(28)

Formule brute : $C_{32}H_{37}F_2N_3O_9S$
Masse moléculaire : 677,70
Cristaux beiges pulvérulents
Point de fusion : 166-168 °C
**RMR** (DMSO $d_6$) δ : 1,52-1,67 (m, 2H) ; 1,69-1,80 (m, 2H) ; 1,91-2,01 (m, 2H) ; 2,42-2,53 (m, 2H) ; 2,62 (t, 2H) ; 2,8 (t, 2H) ; 3,02-3,13 (m, 2H) ; 3,42 (t, 2H) ; 3,55 (t, 2H) ; 3,91 (s, 2H) ; 4,03 (m, 1H) ; 6,58 (m, 4H) ; 6,85-6,97 (m, 2H) ; 7,03-7,2 (m, 2H) ; 7,27-7,38 (m, 2H) ; 7,1-7,8 (m, 1H) ; 11,5-13,5 (m, 4H).

__Exemple 29 :__

**Dihydrogénofumarate de la N-[1-[3-[2-(4-fluorophényl)éthoxy]propyl]pipéridin-4-yl]-4H-3,1-benzothiazin-2-amine (29).**

En opérant comme décrit dans l'exemple précédent, on prépare avec un rendement de 42 % le composé de **formule 29 :**

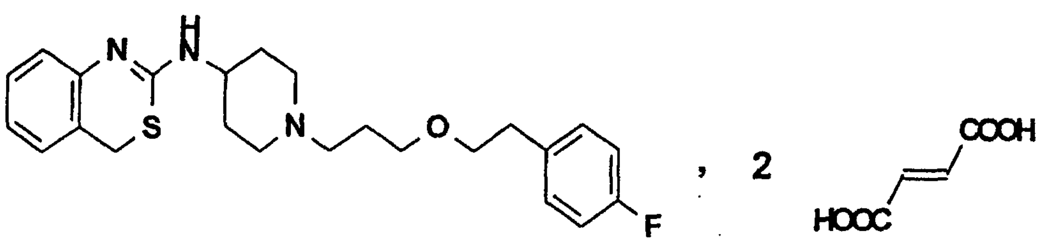

, 2

(29)

Formule brute : $C_{32}H_{38}F_2N_3O_9S$
Masse moléculaire : 678,70
Cristaux crème clair
Point de fusion : 162-163 °C
**RMN** (DMSO $d_6$) δ : 1,52-1,66 (m, 2H) ; 1,68-1,83 (m, 2H) ; 1,94-2,01 (m, 2H) ; 2,4-2,52 (m, 2H) ; 2,61 (t, 2H) ; 2,79 (t, 2H) ; 3,03-3,1 (m, 2H) ; 3,41 (t, 2H) ; 3,55 (t, 2H) ; 3,9 (s, 2H) ; 4,03 (m, 1H) ; 6,58 (s, 4H) ; 6,87-6,97 (m, 2H) ; 7,05-7,19 (m, 4H) ; 7,24-7,3 (m, 2H) ; 7-7,6 (m, 1H) ; 11,8-13 (m, 4H).

**Exemple 30 :**

**Hydrate du dichlorhydrate de la N-[1-[3-[2-(4-fluorophényl)éthoxy]propyl] pipéridin-4-yl]-4H-3,1-benzoxazin-2-amine (30).**

**30.1)** 1-[1-[3-[2-(4-fluorophényl)éthoxy]propyl]pipéridin-4-yl]-3-(2-hydroxyméthyl phényl]urée (**30.1**). Une solution de 0,77 g (2,58 mmol) de triphosgène dans 50 ml de $CH_2Cl_2$ sec est refroidie à 0 °C, puis traitée, goutte à goutte, avec une solution de 2,01 g (7,17 mmol) de 4-amino-1-[3-[2-(4-fluorophényl)éthoxy]propyl]pipéridine (préparée avec un rendement de 52 % comme décrit dans l'exemple **25.3** mais en utilisant de l'acétate d'ammonium comme agent aminant) et de 1,1 ml (7,88 mmol) de triéthylamine dans 20 ml de $CH_2Cl_2$ sec. Le mélange réactionnel est agité une nuit à 25 °C, puis est traité goutte à goutte, avec une solution de 885 mg (7,17 mmol) d'alcool orthoaminobenzylique et 1 ml de triéthylamine dans 25 ml de $CH_2Cl_2$ sec. L'agitation est poursuivie pendant 7 h à 25 °C, puis le mélange réactionnel est lavé à l'eau, à l'eau salée, séché sur sulfate de sodium anhydre et évaporé à siccité. L'huile brune résiduelle est triturée dans l'éther isopropylique pour donner 2,16 g (Rdt : 70 %) de composé de **formule 30.1 :**

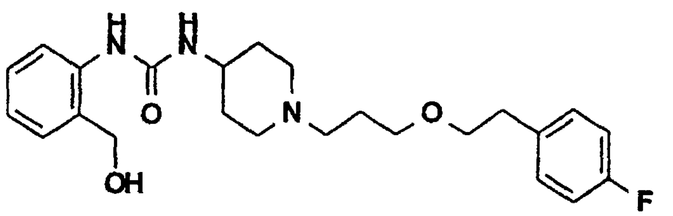

**( 30-1 )**

Formule brute : $C_{24}H_{32}FN_3O_3$
Masse moléculaire : 429,52
Poudre beige foncé
**RMN** ($CDCl_3$) δ : 1,35-1,47 (m, 2H) ; 1,65-1,78 (m, 2H) ; 1,86-1,99 (m,2H) ; 2,05 (t, 2H) ; 2,34 (t, 2H) ; 2,72-2,88 (m, 4H) ; 3,44 (t, 2H) ; 3,55-3,74 (m, 3H) ; 4,66 (s, 2H) ; 5,08 (m, 1H) ; 6,96 (t, 2H) ; 7,02 (t, 1H) ; 7,11-7,21 (m, 3H) ; 7,23-7,3 (m, 2H) ; 7,57 (s, 1H) ; 7,79 (d, 1H).

**30.2)** Hydrate du dichlorhydrate de la N-[1-[3-[2-(4-fluorophényl)éthoxy]propyl] pipéridin-4-yl]-4H-3,1-benzoxazin-2-amine (**30**). En opérant comme décrit dans l'exemple **25.5,** mais en partant de 1,75 g (4,07 mmoles) d'hydroxy urée précédente (**30.1**) on prépare avec un rendement de 61 % le composé de **formule 30 :**

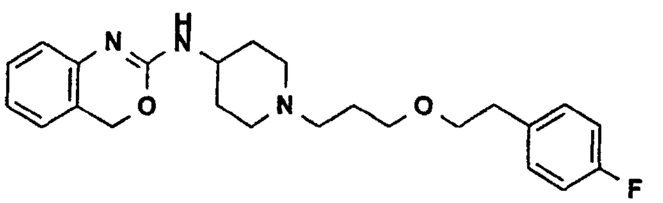

**2 HCl , $H_2O$ ( 30 )**

Formule brute : $C_{24}H_{34}Cl_2FN_3O_3$
Masse moléculaire : 502,49
Cristaux blancs pulvérulents
Point de fusion : 158-159 °C
**RMN** ($CD_3OD$) δ : 1,87-2,4 (m, 6H) ; 2,86 (t, 2H) ; 2,95-3,4 (m, 4H) ; 3,57 (t, 2H) ; 3,67 (t, 4H) ; 4,14 (m, 1H) ; 7,01 (t, 2H) ; 7,12-7,35 (m, 5H) ; 7,39-7,45 (m, 1H).

**EXPERIMENTATIONS BIOLOGIQUES :**

Les composés de la présente invention de formule I et leurs sels thérapeutiquement acceptables présentent

d'intéressantes propriétés pharmacologiques.

Ces dérivés sont actifs ***in vitro*,** au niveau du cardiomyocyte, en inhibant la contracture diastolique induite par la vératrine, au niveau de l'oreillette gauche isolée de rat.

Ils abolissent également la contracture diastolique induite par l'ischémie globale du coeur isolé perfusé de cobaye selon la technique de Langendorff.

Ces composés sont aussi actifs ***in vivo*,** lors de l'ischémie-reperfusion chez le lapin anesthésié : ils inhibent les perturbations électriques de l'ECG provoquées par l'ischémie reperfusion, sans effet hémodynamique notable et ne sont pas dépresseurs cardiaques.

De tels composés sont utiles à titre préventif ou curatif dans le traitement des coronaropathies, de l'ischémie cardiaque et cérébrale sous toutes leurs formes et dans le traitement de l'athérosclérose, de l'insuffisance cardiaque, de la douleur, de la migraine et de l'épilepsie.

**1°) Etude pharmacologique :**

Les expérimentations, auxquelles ont été soumises les molécules chimiques objet de la présente invention, ont permis de mettre en évidence une intéressante activité sur le système cardiovasculaire à la fois sur des tests **"in vitro"** et **"in vivo".**

**a)Action "in vitro"**

1°) L'inibition de la contracture à la vératrine, de l'oreillette gauche isolée de rat, a été réalisée selon la technique de **Le Grand** et coll. (*Naunyn -Schmiedeberg's Arch Pharmacol* (1993) **348** p 184-190). Les résultats sont portés dans le tableau **I**. Les pourcentages d'inhibition sont relatifs à une concentration de $10^{-7}$M en composé étudié.

Tableau I

| Composé n° | Ex.5 | Ex.6 | Ex.14 | Ex.23 | R56865* | Sabeluzole ** | Témoin *** |
|---|---|---|---|---|---|---|---|
| % inhibition à $10^{-7}$M | 63% | 41% | 32% | 35% | 32% | 5% | 26% |

*N-(1-(4-(4-fluorophénoxy)butyl)pipéridin-4-yl)-N-méthyl-2-benzothiazolamine.

**N-(1-(2-hydroxy-3-(4-fluorophénoxy)propyl)pipéridin-4-yl)-N-méthyl-2-benzothiazolamine cités dans le breveet EP 0 184 257 et en développement.

***N-méthyl-N-[1-[4-(3,4-difluorophénoxy)butyl]pipéridin-4-yl]-4H-3,1-benzo-thiazin-2-amine (WO 97-05134 de la demanderesse).

Les composés de la présente invention ne sont pas inotropes négatifs à la concentration de 10 µM.

**2°)** L'inhibition de la contracture diastolique induite par l'ischémie globale du coeur isolé perfusé de cobaye, est réalisée selon la technique décrite par **Le Grand B.** et coll. (*Am. J. Physiol*. **269,** H533-H540 (1995). Les coeurs de cobaye prélevés sont perfusés par une solution modifiée de Krebs et après 20 minutes de stabilisation on ajoute le composé à étudier en solution dans l'eau, ou dans une solution aqueuse à 1% de DMSO. Après 15 minutes l'ischémie globale est provoquée par arrêt de la perfusion coronaire pendant 50 minutes, suivie par la reperfusion pendant 1h. L'inhibition de la contracture ischémique, en présence des composés de la présente invention, est mesurée par rapport à celle obtenue dans le groupe véhicule. Les résultats sont portés dans le **tableau II** à titre d'exemple non limitatif à la concentration de 1 µM.

Tableau II

| Composés | De l'exemple 6 | De l'exemple 14 | Témoin*** F | HOE 694**** |
|---|---|---|---|---|
| % inhibition contracture diastolique à 1 µM | 79% | 77% | 48% | 17% |

*** voir tableau I

**** chlorhydrate de 3-(méthylsulfonyl-4-pipéridinobenzoyl)guanidine (EP418499).

**b)Activité "in vivo"**

Les composés de la présente invention sont aussi actifs par voie orale dans le test de **l'ischémie-reperfusion chez le lapin anesthésié,** selon la méthode de **Verscheure** et coll. *(J Cardiovasc Pharmacol* (1995) **25** p 126-133).

Les résultats pour les composés des exemples **6, 14** sont donnés dans le **tableau III** ci-dessous, à titre d'exemple non limitatif :

Tableau III

| Composé n° | Dose mg/kg p. o. | % inhibition surélévation segment ST | Nombre d'animaux présentant des arythmies de reperfusion | % variation fréquence cardiaque | % variation pression artérielle |
|---|---|---|---|---|---|
| 6<br>14 | 0,63<br>0,63 | 42%<br>83% | 3/5<br>0/5 | -7<br>-5 | 0<br>3 |
| R 56865*<br>"<br>Sabeluzole ** | 10<br>2,5<br>10 | 100%<br>0%<br>34% | 0/5<br>2/5<br>3/4 | 5<br>0<br>0 | 15<br>27<br>12 |
| Témoin*** F | 0,63 | 31% | 1/5 | 0 | 5 |

*, **, *** : voir tableau I.

**2°) Applications thérapeutiques**

Les composés de la présente invention et leurs sels thérapeutiquement acceptables sont utiles comme médicaments.

Ces composés sont plus particulièrement adaptés en cardiologie dans le traitement prophylactique des maladies cardiovasculaires comme :

* l'ischémie du myocarde et les coronaropathies et plus particulièrement dans les crises :
    - d'angor stable chronique,
    - d'angor instable et de Prinzmetal,
* l'ischémie silencieuse, et dans la prévention des réocclusions, resténoses et le réinfarctus.
* l'ischémie cérébrale et plus précisément dans :
    - l'accident vasculaire cérébral,
    - l'attaque ischémique transitoire,
    - les maladies neurodégénératives,
* l'athérosclérose,
* l'insuffisance cardiaque,
* l'hypertension.

Ces composés peuvent également être utilisés dans le traitement de l'épilepsie, de la migraine et de la douleur. L'administration de ces composés peut être réalisée par voie orale, parentérale ou rectale. Chaque dose est constituée d'un adjuvant inerte favorisant la préparation et l'absorption du médicament ; le principe actif pouvant être aussi associé à un autre.

Ces médicaments peuvent se présenter sous forme solide (comprimés ou gélules) ou liquide à réaliser au moment de l'emploi (suspensions, émulsions, sirops, solutions ou autres) ou suppositoires. L'administration du principe actif se fait à la dose moyenne comprise entre 0,1 et 10 mg/kg du poids du corps.

Deux préparations sont données à titre d'exemple non limitatifs. Les ingrédients ainsi que d'autres, thérapeutiquement acceptables peuvent être introduits en d'autres proportions sans modifier la portée de l'invention.

**Exemple 31 :**

Solution injectable à réaliser au moment de l'emploi.

1°) Un flacon stérile pour préparation injectable en verre inactinique renfermant :

| | |
|---|---|
| Hydrogénomaléate de la N-méthyl-N-[1-[3-[2-(3,4-diflurophényl)éthoxy]propyl]pipéridin-4-yl]-4H-3,1-benzothiazin-2-amine | 5 mg |

2°) Une ampoule de solvant en verre, stérile renfermant :

| | |
|---|---|
| Propylène glycol | 100 mg |
| Dextrose anhydre | 50 mg |
| Eau distillée stérile q.s.p. | 2 ml |

**Exemple 32 :**

Comprimés sécables.

| | |
|---|---|
| Hydrogénomaléate de la N-méthyl-N-[1-[3-[2-(3,4-difluorophényl)éthoxy]propyl] pipéridin-4-yl]-4H-3,1-benzothiazin-2-amine | 30 mg |
| Lactose hydrate | 80 mg |
| Cellulose microcristalline | 20 mg |
| Stéarate de magnésium | 4 mg |
| Amidon de maïs | 16 mg |
| Talc | 4 mg |
| Polyvinyl pyrrolidone | 6 mg |
| Poids total | 160 mg |

Comprimés sécables à conserver à l'abri de la chaleur et de l'humidité.

## Revendications

1. N-alcoyl-N-[1-[ω̄-(arylalcoyloxy)alcoyl]-pipéridin-4-yl]-4H-3,1-benzo(thia/oxa)zines-2-amines substituées de **formule I :**

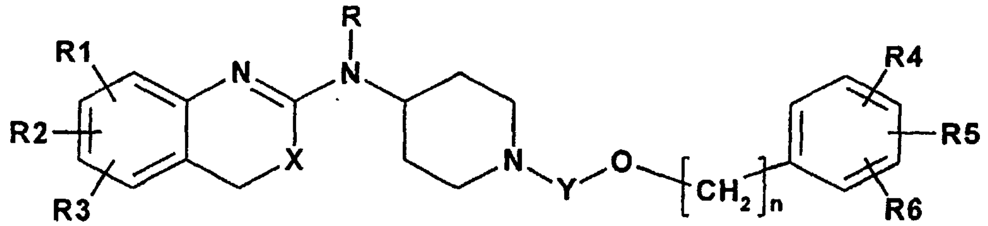

**( I )**

dans laquelle :

- **X** représente un atome d'oxygène ou de soufre,
- **Y** représente soit :

  * un radical alcoylène, ramifié ou non renfermant de 2 à 6 atomes de carbone,
  * un radical - $CH_2$-CH(OH)-$CH_2$-

- **R** représente : un hydrogène, un radical alcoyle saturé ou non, ramifié ou non comportant de 1 à 7 atomes de carbone,
- **$R_1$** à **$R_6$** égaux ou différents représentent :

  * un hydrogène,
  * un alcoyle ramifié ou non, saturé ou non renfermant de 1 à 5 atomes de carbone,
  * un alcoyloxy ramifié ou non saturé ou non renfermant de 1 à 5 atomes de carbone,
  * un groupement halogéno,
  * un groupement nitro,
  * un groupement hydroxy,
  * un groupement acyle ou acyloxy comportant de 2 à 3 atomes de carbone,
  * un groupement dialcoylamino renfermant de 1 à 5 atomes de carbone,

    * un groupement trifluorométhyle ou trifluoro méthoxyle,

- **n** est un nombre entier pouvant varier de 1 à 6 inclus ainsi que leurs énantiomères purs ou leurs mélanges, et les sels minéraux ou organiques thérapeutiquement acceptables des composés de formule I et leurs hydrates éventuels.

**2.** Composé selon la revendication 1 **caractérisé en ce qu'**il est choisi parmi les composés suivants :

Hydrogénofumarate de la N-méthyl-N-[1-[5-(4-fluorobenzyloxy)pentyl]pipéridin-4-yl]-4H-3,1-benzothiazin-2-amine.
Hydrogénofumarate de la N-méthyl-N-[1-[4-(4-fluorobenzyloxy)butyl]pipéridin-4-yl]-4H-3,1-benzothiazin-2-amine.
Hydrogénofumarate de la N-méthyl-N-[1-[3-4(-fluoropenzyloxy)propyl]pipéridin-4-yl]-4H-3,1-benzothiazin-2-amine.
Hydrogénofumarate de la N-méthyl-N-[1-[2-(4-fluorobenzyloxy)éthyl]pipéridin-4-yl]-4H-3,1-benzothiazin-2-amine.
Hydrogénofumarate de la N-méthyl-N-[1-[4-[2-(4-fluorophényl)éthoxy]butyl]pipé-ridin-4-yl]-4H-3,1-benzothiazin-2-amine.
Hydrogénofumarate de la N-méthyl-N-[1-[3-[2-(4-fluorophényl)éthoxy]propyl]pipé-ridin-4-yl]-4H-3,1-benzothiazin-2-aminé.
Dichlorhydrate de la N-méthyl-N-[1-[3-[2-(4-fluorophényl)éthoxy]propyl]pipéridin-4-yl]-4H-3,1-benzothiazin-2-amine.
Hydrogénofumarate de la N-méthyl-N-[1-[3-[3-(4-fluorophenyl)propoxy]propyl] pipéridin-4-yl]-4H-3,1-benzothiazin-2-amine.
Hydrogénofumarate de la N-méthyl-N-[1-[2-[2-(4-fluorophényl)éthoxy]éthyl] pipéridin-4-yl]-4H-3,1-benzotiazin-2-amine.
Hydrogénofumarate de la N-méthyl-N-[1-[5-(3,4-difluorophénylméthoxy)pentyl) pipéridin-4-yl]-4H-3,1-benzothiazin-2-amine.
Hydrogénofumarate de la N-méthyl-N-[1-[4-[3,4-difluorophénylméthoxy)butyl] pipéridin-4-yl]-4H-3,1-benzothiazin-2-amine.
Hydrogénofumarate de la N-méthyl-N-[1-[3-(3,4-difluorophénylméthoxy)propyl] pipéridin-4-yl]-4H-3,1-benzothiazin-2-amine.
Hydrogénofumarate de la N-méthyl-N-[1-[4-[2-(3,4-difluorophényl)éthoxy]butyl] pipéridin-4-yl]-4H-3,1-benzothiazin-2-amine.
Hydrogénofumarate de la N-méthyl-N-[1-[3-[2-(3,4-difluorophényl)éthoxy]propyl] pipéridin-4-yl]-4H-3,1-benzothiazin-2-amine.
Hydrogénofumarate de la N-méthyl-N-[1-[5-(4-méthoxyphénylméthoxy)pentyl] pipéridin-4-yl]-4H-3,1-benzothiazin-2-amine.
Hydrogénofumarate de la N-méthyl-N-[1-[4-(4-méthoxyphénylméthoxy)butyl]pipé-ridin-4-yl]-4H-3,1-benzo-

thiazin-L-amine.
Hydrogénofumarate de la N-méthyl-N[1-[3-[4-méthoxyphénylméthoxy)propyl]pipé-ridin-4-yl]-4H-3,1-benzothiazin-2-amine.
Hydrogénofumarate de la N-méthyl-N-[1-[4-[2-(4-méthoxyphényl)éthoxy]butyl] pipéridin-4-yl]-4H-3,1-benzothiazin-2-amine.
Hydrogénofumarate de la N-méthyl-N-[1-[3-[2-(4-méthoxyphényl)éthoxy]propyl] pipéridin-4-yl]-4H-3,1-benzothiazin-2-amine.
Hydrogénofumarate de la N-méthyl-N-[1-[2-[2-(4-méthoxyphényl)éthoxy]éthyl] pipéridin-4-yl]-4H-3,1-benzothiazin-2-amine.
Hydrogénofumarate de la N-méthyl-N-[1-(2-phénylméthoxyéthyl)pipéridin-4-yl]-4H-3,1-benzothiazin-2-amine.
Hydrogénofumarate de la N-méthyl-N-[1-[3-(2-phényléthoxy) propyl]pipéridin-4-yl]-4H-3,1-benzothiazin-2-amine.
Hémifumarate de la N-méthyl-N-[1-[2-hydroxy-3-(4-fluorophénylméthoxy)propyl] pipéridin-4-yl]-4H-3,1-benzothiazin-2-amine.
Hydrogénofumarate de la N-méthyl-N-[1-[2-hydroxy-3-[2-(4-méthoxyphényl) éthoxy]propyl]pipéridin-4-yl]-4H-3,1-benzothiazin-2-amine.
Hydrogénofumarate de la 6,N-diméthyl-N-[1- [3-[2-(4-fluorophenyl)éthoxy]propyl] pipéridin-4-yl]-4H-3,1-benzothiazin-2-amine.
Hydrogénofumarate de la 6-chloro-N-méthyl-N-[1-[3-[2- (4-fluorophényl)éthoxy] propyl]pipéridin-4-yl]-4H-3,1-benzothiazin-2-amine.
Hydrogénofumarate de la N-méthyl-N-[1-[3-[2-(4-fluorophényl)éthoxy]propyl]pipé-ridin-4-yl]-6,7,8-triméthoxy-4H-3,1-benzothiazin-2-amine.
Dihydrogénofumarate de la N-[1-[3-[2-(4-fluorophényl) éthoxy]propyl]pipéridin- 4yl]-4H-3,1-benzothiazin-2-amine.
Dihydrogénofumarate de la N-[1-3-[2-(4-fluorophényl) éthoxy]propyl]pipéridin-4-yl] -4H-3,1-benzothiazin-2-amine.
Hydrate du dichlorhydrate de la N-[1-[3-[2-(4-fluorophényl) éthoxy]propyl]pipéridin-4- yl]-4H-3,1-benzoxazin-2-amine.
N-méthyl-N-[1-[3-[2-(4-fluorophényl)éthoxy]propyl]pipéridin-4-yl]-4H-3,1-benzothia-zin-2-amine.
N-méthyl-N-[1-[3-[3-(4-fluorophenyl)propoxy]propyl] pipéridin-4-yl]-4H-3,1-benzo-thiazin-2-amine.
N-méthyl-N-[1-[3-[2-(3,4-difluorophényl)éthoxy]propyl]pipéridin-4-yl]-4H-3,1-benzo thiazin-2-amine.
N-méthyl-N-[1-[3-(2-phényléthoxy)propyl]pipéridin-4-yl]-4H-3,1-benzothiazin-2-amine.
6,N-diméthyl-N-[1-[3-[2-(4-fluorophenyl)éthoxy]propyl] pipéridin-4-yl]-4H-3,1-benzo-thiazin-2-amine.
6-chloro-N-méthyl-N-[1-[3-[2-(4-fluorophényl)éthoxy]propyl] pipéridin-4-yl]-4H-3,1-benzothiazin-2-amine.
N-méthyl-N-[1-[3-[2-(4-fluorophényl)éthoxy]propyl]pipéridin -4-yl]-6,7,8-triméthoxy -4H-3,1-benzothiazin-2-amine.

3. Procédé de préparation des composés chimiques de formule **I** selon les revendications 1 et 2, **caractérisé en ce que** l'on active l'amine intermédiaire (**X**) :

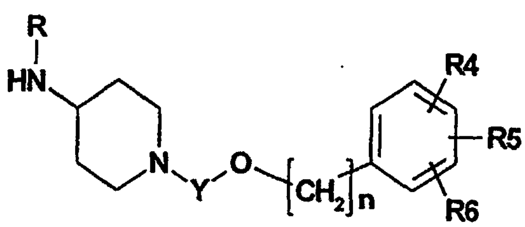

(X)

sous forme de chlorure de carbamoyle ou de thio carbamoyle de **formule XIV :**

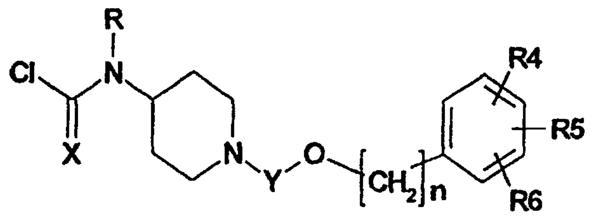

(XIV)

Cet intermédiaire qui n'est pas isolé, peut être préparé par réaction du (thio)phosgène sur l'amine **X** en présence de base, dans un solvant aprotique halogéné ($CH_2Cl_2$, $C_2H_4Cl_2$) ou un éther. Il est ensuite condensé sur un alcool o-aminobenzylique de **formule XII :**

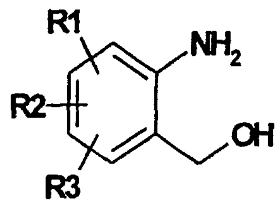

(XII)

dans le solvant précédent à une température comprise entre 10 °C et 40 °C pour donner l'hydroxyméthyl(thio) urée intermédiaire **XV,** généralement récupérée par cristallisation :

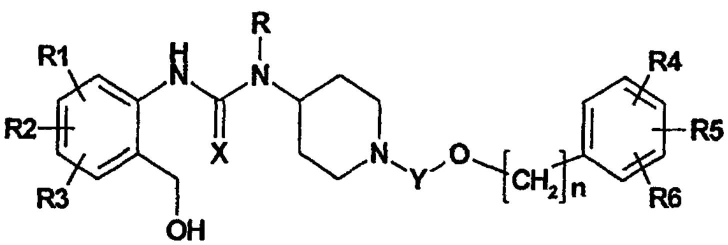

(XV)

Celle-ci est facilement cyclisée dans une solution aqueuse concentrée d'hydracide à une température comprise entre 20 et 80 °C, pour donner le composé de formule générale (**I**) .
Dans les formules **X, XII, XIV, XV,** les radicaux, **R, $R_1$**à **$R_6$, n, X, Y** ont la même signification que dans **I.**

**4.** A titre de médicaments nouveaux les composés définis selon l'une des revendications 1 et 2.

**5.** Composition pharmaceutique, **caractérisée en ce qu'**elle contient comme principe actif au moins un composé selon l'une des revendications 1, 2 et 4, associé à un support pharmaceutique inerte, ou autres véhicules pharmaceutiquement acceptables et pouvant ou non être associé à un autre médicament.

**6.** Composition pharmaceutique selon la revendication 5 utile dans le traitement symptomatique et prophylactique de l'ischémie myocardique comme les crises d'angor stable chronique, l'angor instable et de Prinzmetal, l'ischémie cardiaque silencieuse, le réinfarctus, la réocclusion et la resténose.

**7.** Composition pharmaceutique selon la revendication 5 utile dans l'ischémie cérébrale, l'accident vasculaire cérébral, l'attaque ischémique transitoire et les maladies neurodégénératives.

**8.** Composition pharmaceutique selon la revendication 5 **caractérisée en ce qu'**elle permet de prévenir et de traiter l'athérosclérose.

**9.** Composition pharmaceutique selon la revendication 5 **caractérisée en ce qu'**elle permet de traiter l'insuffisance cardiaque et l'hypertension.

**10.** Composition pharmaceutique selon la revendication 5 **caractérisée en ce qu'**elle permet de traiter l'épilepsie, la migraine et la douleur.

**Patentansprüche**

**1.** Substituierte N-Alkyl-N-[1-[1-ω-(arylalkyloxy)alkyl]piperidin-4-yl]-4H-3,1-benzo(thia/oxa)zin-2-amine der Formel I:

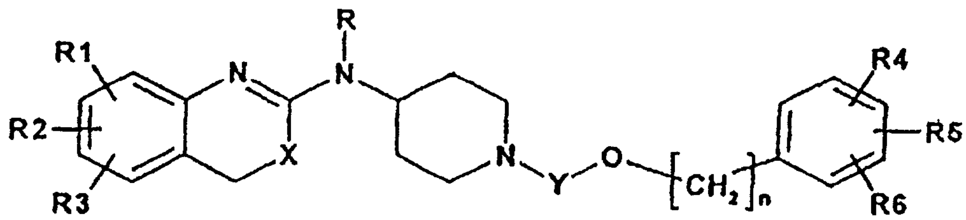

(I)

in der:

- X ein Sauerstoff- oder Schwefelatom darstellt,
- Y entweder
    * einen verzweigten oder unverzweigten Alkylenrest, der 2 bis 6 Kohlenstoffatome einschließt,
    * einen Rest $-CH_2-CH(OH)-CH_2-$

  darstellt,
- R einen Wasserstoff, einen gesättigten oder ungesättigten, verzweigten oder unverzweigten Alkylrest, der 1 bis 7 Kohlenstoffatomen umfasst, darstellt,
- $R_1$ bis $R_6$, gleich oder verschieden,
    * einen Wasserstoff,
    * ein verzweigtes oder unverzweigtes, gesättigtes oder ungesättigtes Alkyl, das 1 bis 5 Kohlenstoffatome einschließt,
    * ein verzweigtes oder unverzweigtes, gesättigtes oder ungesättigtes Alkyloxy, das 1 bis 5 Kohlenstoffatome einschließt,
    * eine Halogengruppe,
    * eine Nitrogruppe,
    * eine Hydroxygruppe,
    * eine Acyl- oder Acyloxygruppe, die 2 bis 3 Kohlenstoffatome umfasst,
    * eine Dialkylaminogruppe, die 1 bis 5 Kohlenstoffatome einschließt,
    * eine Trifluormethyl- oder Trifluormethoxygruppe

  darstellt,
- n eine ganze Zahl ist, die von 1 bis 6 einschließlich variieren kann,

sowie deren reine Enantiomere oder deren Mischungen und die therapeutisch annehmbaren Mineral- oder organischen Salze der Verbindungen der Formel I und gegebenenfalls ihre Hydrate.

**2.** Verbindung nach Anspruch 1, **dadurch gekennzeichnet, dass** sie aus den folgenden Verbindungen ausgewählt ist:

N-Methyl-N-[1-[5-(4-fluorbenzyloxy)pentyl]piperidin-4-yl]-4H-3,1-benzothiazin-2-amin-Hydrogenfumarat.
N-Methyl-N-[1-[4-(4-fluorbenzyloxy)butyl]piperidin-4-yl]-4H-3,1-benzothiazin-2-amin-Hydrogenfumarat.
N-Methyl-N-[1-[3-(4-fluorbenzyloxy)propyl]piperidin-4-yl]-4H-3,1-benzothiazin-2-amin-Hydrogenfumarat.

N-Methyl-N-[1-[2-(4-fluorbenzyloxy)ethyl]piperidin-4-yl]-4H-3,1-benzothiazin-2-amin-Hydrogenfumarat.
N-Methyl-N-[1-[4-[2-(4-fluorphenyl)ethoxy]butyl]piperidin-4-yl]-4H-3,1-benzothiazin-2-amin-Hydrogenfumarat.
N-Methyl-N-[1-[3-[2-(4-fluorphenyl)ethoxy]propyl]piperidin-4-yl]-4H-3,1-benzothiazin-2-amin-Hydrogenfumarat.
N-Methyl-N-[1-[3-[2-(4-fluorphenyl)ethoxy]propyl]piperidin-4-yl]-4H-3,1-benzothiazin-2-amin-Dihydrochlorid.
N-Methyl-N-[1-[3-[3-(4-fluorphenyl)propoxy]propyl]piperidin-4-yl]-4H-3,1-benzothiazin-2-amin-Hydrogenfumarat.
N-Methyl-N-[1-[2-[2-(4-fluorphenyl)ethoxy]ethyl]piperidin-4-yl]-4H-3,1-benzothiazin-2-amin-Hydrogenfumarat.
N-Methyl-N-[1-[5-(3,4-difluorphenylmethoxy)pentyl]piperidin-4-yl]-4H-3,1-benzothiazin-2-amin-Hydrogenfumarat.
N-Methyl-N-[1-[4-(3,4-difluorphenylmethoxy)butyl]piperidin-4-yl]-4H-3,1-benzothiazin-2-amin-Hydrogenfumarat.
N-Methyl-N-[1-[3-(3,4-difluorphenylmethoxy)propyl]piperidin-4-yl]-4H-3,1-benzothiazin-2-amin-Hydrogenfumarat.
N-Methyl-N-[1-[4-[2-(3,4-difluorphenyl)ethoxy]butyl]piperidin-4-yl]-4H-3,1-benzothiazin-2-amin-Hydrogenfumarat.
N-Methyl-N-[1-[3-[2-(3,4-difluorphenyl)ethoxy]propyl]piperidin-4-yl]-4H-3,1-benzothiazin-2-amin-Hydrogenfumarat.
N-Methyl-N-[1-[5-(4-methoxyphenylmethoxy)pentyl]piperidin-4-yl]-4H-3,1-benzothiazin-2-amin-Hydrogenfumarat.
N-Methyl-N-[1-[4-(4-methoxyphenylmethoxy)butyl]piperidin-4-yl]-4H-3,1-benzothiazin-L-amin-Hydrogenfumarat.
N-Methyl-N-[1-[3-(4-methoxyphenylmethoxy)propyl]piperidin-4-yl]-4H-3,1-benzothiazin-2-amin-Hydrogenfumarat.
N-Methyl-N-[1-[4-[2-(4-methoxyphenyl)ethoxy]butyl]piperidin-4-yl]-4H-3,1-benzothiazin-2-amin-Hydrogenfumarat.
N-Methyl-N-[1-[3-[2-(4-methoxyphenyl)ethoxy]propyl]piperidin-4-yl]-4H-3,1-benzothiazin-2-amin-Hydrogenfumarat.
N-Methyl-N-[1-[2-[2-(4-methoxyphenyl)ethoxy]ethyl]piperidin-4-yl]-4H-3,1-benzothiazin-2-amin-Hydrogenfumarat.
N-Methyl-N-[1-(2-phenylmethoxyethyl)piperidin-4-yl]-4H-3,1-benzothiazin-2-amin-Hydrogenfumarat.
N-M ethyl-N-[1-[3-(2-phenylethoxy)propyl]piperidin-4-yl]-4H-3,1-benzothiazin-2-amin-Hydrogenfumarat.
N-Methyl-N-[1-[2-hydroxy-3-(4-fluorphenylmethoxy)propyl]piperidin-4-yl]-4H-3,1-benzothiazin-2-amin-Hemifumarat.
N-Methyl-N-[1-[2-hydroxy-3-[2-(4-methoxyphenyl)ethoxy]propyl]piperidin-4-yl]-4H-3,1-benzothiazin-2-amin-Hydrogenfumarat.
6,N-Dimethyl-N-[1-[3-[2-(4-fluorphenyl)ethoxy]propyl]piperidin-4-yl]-4H-3,1-benzothiazin-2-amin-Hydrogenfumarat.
6-Chlor-N-methyl-N-[1-[3-[2-(4-fluorphenyl)ethoxy]propyl]piperidin-4-yl]-4H-3,1-benzothiazin-2-amin-Hydrogenfumarat.
N-Methyl-N-[1-[3-[2-(4-fluorphenyl)ethoxy]propyl]piperidin-4-yl]-6,7,8-trimethoxy-4H-3,1-benzothiazin-2-amin-Hydrogenfumarat.
N-[1-[3-[2-(4-Fluorphenyl)ethoxy]propyl]piperidin-4-yl]-4H-3,1-benzothiazin-2-amin-Dihydrogenfumarat.
N-[1-[3-[2-(4-Fluorphenyl)ethoxy]propyl]piperidin-4-yl]-4H-3,1-benzoxazin-2-amin-Dihydrochloridhydrat.
N-Methyl-N-[1-[3-[2-(4-fluorphenyl)ethoxy]propyl]piperidin-4-yl]-4H-3,1-benzothiazin-2-amin.
N-Methyl-N-[1-[3-[3-(4-fluorphenyl)propyloxy]propyl]piperidin-4-yl]-4H-3,1-benzothiazin-2-amin.
N-Methyl-N-[1-[3-[2-(3,4-difluorphenyl)ethoxy]propyl]piperidin-4-yl]-4H-3,1-benzothiazin-2-amin.
N-Methyl-N-[1-[3-(2-phenylethoxy)propyl]piperidin-4-yl]-4H-3,1-benzothiazin-2-amin.
6,N-Dimethyl-N-[1-[3-[2-(4-fluorphenyl)ethoxy]propyl]piperidin-4-yl]-4H-3,1-benzothiazin-2-amin.
6-Chlor-N-methyl-N-[1-[3-[2-(4-fluorphenyl)ethoxy]propyl]piperidin-4-yl]-4H-3,1-benzothiazin-2-amin.
N-Methyl-N-[1-[3-[2-(4-fluorphenyl)ethoxy]propyl]piperidin-4-yl]-6,7,8-trimethoxy-4H-3,1-benzothiazin-2-amin.

3. Verfahren zur Herstellung der chemischen Verbindungen der Formel I gemäß den Ansprüchen 1 und 2, **dadurch gekennzeichnet, dass** man das intermediäre Amin (X):

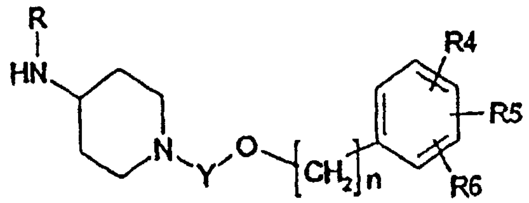

(X)

in Form des Carbamoyl- oder Thiocarbamoylchlorids der Formel XIV:

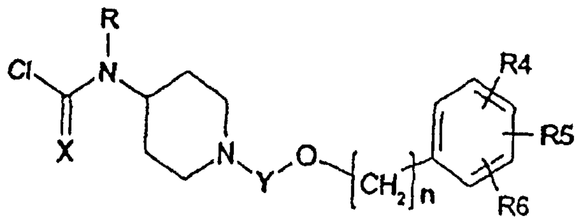

(XIV)

aktiviert, wobei dieses Zwischenprodukt, das nicht isoliert wird, durch Umsetzung von (Thio)phosgen mit dem Amin X in Anwesenheit von Base in einem aprotischen halogenierten Lösungsmittel ($CH_2Cl_2$, $C_2H_4Cl_2$) oder einem Ether hergestellt werden kann, und anschließend mit einem o-Aminobenzylalkohol der Formel XII:

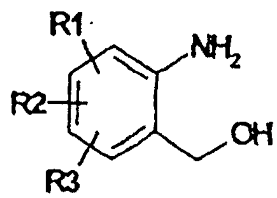

(XII)

in dem vorangehenden Lösungsmittel bei einer Temperatur von 10°C bis 40°C kondensiert, um den intermediären Hydroxymethyl(thio)harnstoff XV zu ergeben, der im Allgemeinen durch Kristallisation gewonnen wird:

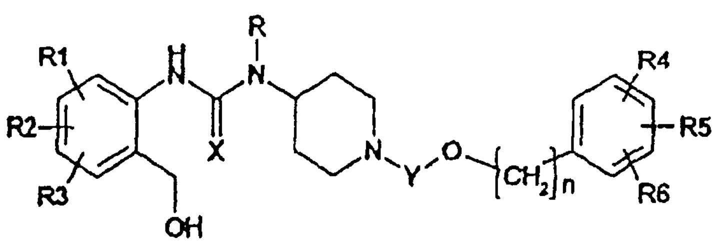

(XV)

wobei dieser leicht in einer konzentrierten wässrigen Wasserstoffsäure-Lösung bei einer Temperatur von 20 bis 80°C zyklisiert wird, um die Verbindung der allgemeinen Formel (I) zu ergeben, wobei in den Formeln X, XII, XIV, XV die Reste R, $R_1$ bis $R_6$, n, X, Y die gleiche Bedeutung wie in I aufweisen.

**4.** Verbindungen, definiert nach einem der Ansprüche 1 und 2, als neue Medikamente.

**5.** Pharmazeutische Zusammensetzung, **dadurch gekennzeichnet, dass** sie als Wirkstoff mindestens eine Verbindung gemäß einem der Ansprüche 1, 2 und 4 zusammen mit einem inerten pharmazeutischen Träger oder anderen pharmazeutisch annehmbaren Vehikeln enthält und mit einem anderen Medikament verbunden sein kann oder

nicht.

6. Pharmazeutische Zusammensetzung nach Anspruch 5, die bei der symptomatischen und prophylaktischen Behandlung von Myokardischämie, wie den Krisen von chronischer stabiler Herzbränne, instabiler und Prinzmetal-Herzbränne, stummer Herzischämie, Reinfarkt, Reokklusion und Restenose, nützlich ist.

7. Pharmazeutische Zusammensetzung nach Anspruch 5, die bei zerebraler Ischämie, zerebraler vaskulärer Erkrankung, vorübergehender ischämischer Attacke und neurodegenerativen Erkrankungen nützlich ist.

8. Pharmazeutische Zusammensetzung nach Anspruch 5, **dadurch gekennzeichnet, dass** sie es gestattet, Atherosklerose zu verhüten und zu behandeln.

9. Pharmazeutische Zusammensetzung nach Anspruch 5, **dadurch gekennzeichnet, dass** sie es gestattet, Herzinsuffizienz und Hochdruck zu behandeln.

10. Pharmazeutische Zusammensetzung nach Anspruch 5, **dadurch gekennzeichnet, dass** sie es gestattet, Epilepsie, Migräne und Schmerz zu behandeln.

## Claims

1. Substituted N-alkyl-N-[1-[ω-(arylalkyloxy)alkyl]-4-piperidyl]-4H-3,1-benzo(thia/oxa)zine-2-amine of **formula I:**

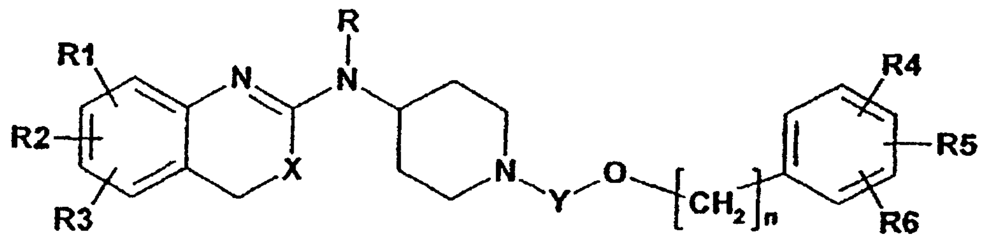

**( I )**

in which:

- **X** represents an oxygen or sulphur atom,
- **Y** represents either:
  - * a branched or unbranched alkylene radical containing from 2 to 6 carbon atoms or
  - * a $-CH_2-CH(OH)-CH_2-$ radical
- **R** represents: a hydrogen, a saturated or unsaturated, branched or unbranched alkyl radical containing from 1 to 7 carbon atoms,
- **$R_1$ to $R_6$,** which may be identical or different, represent:
  - * a hydrogen,
  - * a branched or unbranched, saturated or unsaturated alkyl containing from 1 to 5 carbon atoms,
  - * a branched or unbranched, saturated or unsaturated alkyloxy containing from 1 to 5 carbon atoms,
  - * a halo group,
  - * a nitro group,
  - * a hydroxyl group,
  - * an acyl or acyloxy group containing from 2 to 3 carbon atoms,
  - * a dialkylamino group containing from 1 to 5 carbon atoms,
  - * a trifluoromethyl or trifluoromethoxy group,

- **n** is an integer which may range from 1 to 6 inclusive,

and also the pure enantiomers thereof or mixtures thereof, and the therapeutically acceptable mineral or organic salts of the compounds of formula **I** and the possible hydrates thereof.

**2.** Compound according to Claim 1, **characterized in that** it is chosen from the following compounds:

N-Methyl-N-[1-[5-(4-fluorobenzyloxy)pentyl]-4-piperidyl]-4H-3,1-benzothiazin-2-amine hydrogen fumarate

N-Methyl-N-[1-[4-(4-fluorobenzyloxy)butyl]-4-piperidyl]-4H-3,1-benzothiazin-2-amine hydrogen fumarate

N-Methyl-N-[1-[3-(4-fluorobenzyloxy)propyl]-4-piperidyl]-4H-3,1-benzothiazin-2-amine hydrogen fumarate

N-Methyl-N-[1-[2-(4-fluorobenzyloxy)ethyl]-4-piperidyl]-4H-3,1-benzothiazin-2-amine hydrogen fumarate

N-Methyl-N-[1-[4-[2-(4-fluorophenyl)ethoxy]butyl]-4-piperidyl]-4H-3,1-benzothiazin-2-amine hydrogen fumarate

N-Methyl-N-[1-[3-[2-(4-fluorophenyl)ethoxy]propyl]-4-piperidyl]-4H-3,1-benzothiazin-2-amine hydrogen fumarate

N-Methyl-N-[1-[3-[2-(4-fluorophenyl)ethoxy]propyl]-4-piperidyl]-4H-3,1-benzothiazin-2-amine dihydrochloride

N-Methyl-N-[1-[3-[3-(4-fluorophenyl)propoxy]propyl]-4-piperidyl]-4H-3,1-benzothiazin-2-amine hydrogen fumarate

N-Methyl-N-[1-[2-[2-(4-fluorophenyl)ethoxy]ethyl]-4-piperidyl]-4H-3,1-benzothiazin-2-amine hydrogen fumarate

N-Methyl-N-[1-[5-(3,4-difluorophenylmethoxy)pentyl)-4-piperidyl]-4H-3,1-benzothiazin-2-amine hydrogen fumarate

N-Methyl-N-[1-[4-[3,4-difluorophenylmethoxy)butyl]-4-piperidyl]-4H-3,1-benzothiazin-2-amine hydrogen fumarate

N-Methyl-N-[1-[3-(3,4-difluorophenylmethoxy)propyl]-4-piperidyl]-4H-3,1-benzothiazin-2-amine hydrogen fumarate

N-Methyl-N-[1-[4-[2-(3,4-difluorophenyl)ethoxy]butyl]-4-piperidyl]-4H-3,1-benzothiazin-2-amine hydrogen fumarate

N-Methyl-N-[1-[3-[2-(3,4-difluorophenyl)ethoxy]propyl]-4-piperidyl]-4H-3,1-benzothiazin-2-amine hydrogen fumarate

N-Methyl-N-[1-[5-(4-methoxyphenylmethoxy)pentyl]-4-piperidyl]-4H-3,1-benzothiazin-2-amine hydrogen fumarate

N-Methyl-N-[1-[4-(4-methoxyphenylmethoxy)butyl]-4-piperidyl]-4H-3,1-benzothiazin-L-amine hydrogen fumarate

N-Methyl-N-[1-[3-[4-methoxyphenylmethoxy)propyl]-4-piperidyl]-4H-3,1-benzothiazin-2-amine hydrogen fumarate

N-Methyl-N-[1-[4-[2-(4-methoxyphenyl)ethoxy]butyl]-4-piperidyl]-4H-3,1-benzothiazin-2-amine hydrogen fumarate

N-Methyl-N-[1-[3-[2-(4-methoxyphenyl)ethoxy]propyl]-4-piperidyl]-4H-3,1-benzothiazin-2-amine hydrogen fumarate

N-Methyl-N-[1-[2-[2-(4-methoxyphenyl)ethoxy]ethyl]-4-piperidyl]-4H-3,1-benzothiazin-2-amine hydrogen fumarate

N-Methyl-N-[1-(2-phenylmethoxyethyl)-4-piperidyl]-4H-3,1-benzothiazin-2-amine hydrogen fumarate

N-Methyl-N-[1-[3-(2-phenylethoxy)propyl]-4-piperidyl]-4H-3,1-benzothiazin-2-amine hydrogen fumarate

N-Methyl-N-[1-[2-hydroxy-3-(4-fluorophenylmethoxy)propyl]-4-piperidyl]-4H-3,1-benzothiazin-2-amine hemifumarate

N-Methyl-N-[1-[2-hydroxy-3-[2-(4-methoxyphenyl)-ethoxy]propyl]-4-piperidyl]-4H-3,1-benzothiazin-2-amine hydrogen fumarate

6,N-Dimethyl-N-[1-[3-[2-(4-fluorophenyl)ethoxy]propyl]-4-piperidyl]-4H-3,1-benzothiazin-2-amine hydrogen fumarate

6-Chloro-N-methyl-N-[1-[3-[2-(4-fluorophenyl)ethoxy]propyl]-4-piperidyl]-4H-3,1-benzothiazin-2-amine hydrogen fumarate

N-Methyl-N-[1-[3-[2-(4-fluorophenyl)ethoxy]propyl]-4-piperidyl]-6,7,8-trimethoxy-4H-3,1-benzothiazin-2-amine hydrogen fumarate

N-[1-[3-[2-(4-fluorophenyl)ethoxy]propyl]-4-piperidyl]-4H-3,1-benzothiazin-2-amine dihydrogen fumarate

N-[1-[3-[2-(4-fluorophenyl)ethoxy]propyl]-4-piperidyl]-4H-3,1-benzothiazin-2-amine dihydrogen fumarate

N-[1-[3-[2-(4-fluorophenyl)ethoxy]propyl]-4-piperidyl]-4H-3,1-benzoxazin-2-amine dihydrochloride hydrate

N-Methyl-N-[1-[3-[2-(4-fluorophenyl)ethoxy]propyl]-4-piperidyl]-4H-3,1-benzothiazin-2-amine

N-Methyl-N-[1-[3-[3-(4-fluorophenyl)propoxy]propyl]-4-piperidyl]-4H-3,1-benzothiazin-2-amine

N-Methyl-N-[1-[3-[2-(3,4-difluorophenyl)ethoxy]propyl]-4-piperidyl]-4H-3,1-benzothiazin-2-amine

N-Methyl-N-[1-[3-(2-phenylethoxy)propyl]-4-piperidyl]-4H-3,1-benzothiazin-2-amine

6,N-Dimethyl-N-[1-[3-[2-(4-fluorophenyl)ethoxy]propyl]-4-piperidyl]-4H-3,1-benzothiazin-2-amine

6-Chloro-N-methyl-N-[1-[3-[2-(4-fluorophenyl)ethoxy]propyl]-4-piperidyl]-4H-3,1-benzothiazin-2-amine

N-Methyl-N-[1-[3-[2-(4-fluorophenyl)ethoxy]propyl]-4-piperidyl]-6,7,8-trimethoxy-4H-3,1-benzothiazin-2-amine.

3. Process for preparing a chemical compound of formula **I** according to Claims 1 and 2, **characterized in that** the intermediate amine (**X**):

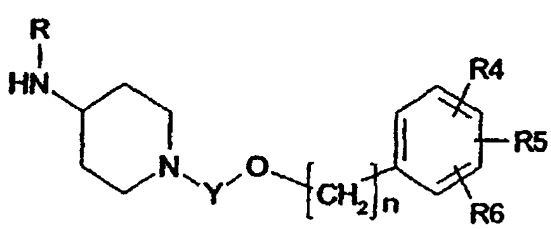

(X)

is activated in the form of the carbamoyl chloride or thiocarbamoyl chloride of **formula XIV:**

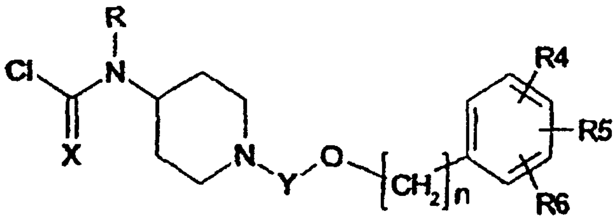

(XIV)

this intermediate, which is not isolated, may be prepared by reacting (thio)phosgene with the amine **X** in the presence of a base, in an aprotic halogenated solvent ($CH_2Cl_2$ or $C_2H_4Cl_2$) or an ether. It is then condensed with an o-aminobenzyl alcohol of **formula XII:**

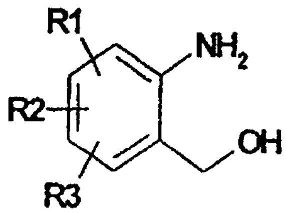

(XII)

in the above solvent at a temperature of between 10°C and 40°C to give the intermediate hydroxymethyl(thio)urea **XV**, which is generally recovered by recrystallization:

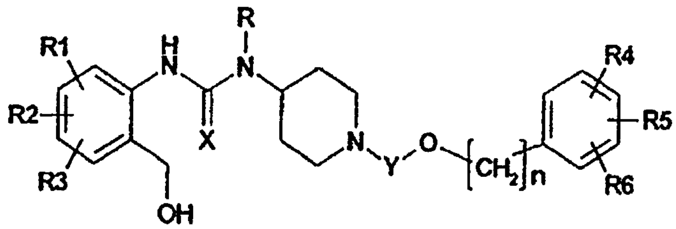

(XV)

This product is readily cyclized in a concentrated aqueous hydracid solution at a temperature between 20 and 80°C, to give the compound of general formula (**I**).
In formulae **X, XII, XIV** and **XV,** the radicals **R,** $\mathbf{R_1}$ to $\mathbf{R_6}$**, n, X** and **Y** have the same meaning as in **I.**

4. As novel medicinal products, the compounds defined in either of Claims 1 and 2.

5. Pharmaceutical composition, **characterized in that** it contains as active principle at least one compound according to one of Claims 1, 2 and 4, combined with an inert pharmaceutical support, or other pharmaceutically acceptable vehicles, which may or may not be combined with another medicinal product.

6. Pharmaceutical composition according to Claim 5, which is useful in the symptomatic and prophylactic treatment of myocardial ischaemia, for instance chronic stable angina, unstable angina and Prinzmetal's angina attacks, silent cardiac ischaemia, reinfarction, reocclusion and restenosis.

7. Pharmaceutical composition according to Claim 5, which is useful in cerebral ischaemia, cerebrovascular accidents, transient ischaemic attacks and neurodegenerative diseases.

8. Pharmaceutical composition according to Claim 5, **characterized in that** it makes it possible to prevent and treat artherosclerosis.

9. Pharmaceutical composition according to Claim 5, **characterized in that** it makes it possible to treat cardiac insufficiency and hypertension.

10. Pharmaceutical composition according to Claim 5, **characterized in that** it makes it possible to treat epilepsy,

migraine and pain.